# NEUE EUROPÄISCHE PATENTSCHRIFT

(11) **EP 1 765 303 B2**
(45) Veröffentlichungstag und Bekanntmachung des Hinweises auf die Entscheidung über den Einspruch: **23.11.2022**
(45) Hinweis auf die Patenterteilung: 20.02.2013
(21) Anmeldenummer: 05769988.6
(22) Anmeldetag: 29.06.2005
(51) Int. Cl.: A61K 9/20, A61K 31/485

(54) **GEGEN MISSBRAUCH GESICHERTE, ORAL TABLETTE**
ORAL TABLET SAFEGUARDED AGAINST ABUSE
COMPRIME ANTI-ABUS POUR ADMINISTRATION PAR VOIE ORALE

(30) Priorität: 01.07.2004 DE 102004032049; 14.07.2004 US 890763
(43) Veröffentlichungstag der Anmeldung: 28.03.2007
(73) Patentinhaber: Grünenthal GmbH, 52078 Aachen (DE)
(72) Erfinder: BARTHOLOMÄUS, Johannes, 52080 Aachen (DE); KUGELMANN, Heinrich, 52068 Aachen (DE); ARKENAU-MARIC, Elisabeth, 50931 Köln (DE)
(74) Vertreter: Kutzenberger Wolff & Partner
(86) Internationale Anmeldenummer: PCT/EP2005/006984
(87) Internationale Veröffentlichungsnummer: WO 2006/002884

(56) Entgegenhaltungen:
- WO-A-97/33566
- WO-A-2004/037230
- WO-A-2004/037259
- WO-A-2004/037260
- WO-A1-2005/016313
- WO-A1-2005/016314
- DE-A1- 10 250 083
- US-A1- 2003 068 392
- US-A1- 2003 068 392
- US-A1- 2003 124 185
- US-B1- 6 488 963

## Beschreibung

Die vorliegende Erfindung betrifft eine gegen Missbrauch gesicherte, orale Darreichungsform mit kontrollierter Opioid-Freisetzung für eine einmal tägliche Verabreichung, die wenigstens ein Opioid mit Missbrauchspotential (A), mindestens ein synthetisches oder natürliches Polymer (C), ggf. retardierende Matrix-Materialien, ggf. wenigstens einen retardierenden Überzug, ggf. wenigsten physiologisch verträgliche Hilfsstoffe (B), ggf. ein Wachs (D) umfasst, wobei die Darreichungsform eine Bruchfestigkeit von mindestens 500 N, vorzugsweise 750 N, aufweist.

Unter der Bezeichnung Opioide werden erfindungsgemäß Verbindungen verstanden, die mit mindestens einem Opioid-Rezeptor in Wechselwirkung treten. Insbesondere Opioide mit Ausnahme von (1 R, 2R)-3-(3-dimethylamino-1-ethyl-2-methyl-propyl)-phenol, dessen physiologisch verträglichen Verbindungen wie Salze und/oder dessen Derivate, sowie die entsprechenden Stereoisomere und/oder pharmazeutisch akzeptablen Verbindungen oder Derivate werden als diejenigen opioide Verbindungen verstanden, die ein Missbrauchspotential aufweisen.

Vorzugsweise werden Opioide zur Schmerzbekämpfung eingesetzt. Dazu werden Analgetika häufig in Langzeittherapien eingesetzt, beispielsweise bei tumorbedingten oder chronischen Schmerzen. Insbesondere bei einer Langzeittherapie ist es wichtig, dem Patienten eine gute Lebensqualität zu ermöglichen. Zu den Maßnahmen, die die Lebensqualität eines Patienten erhöhen, gehören u. a. Darreichungsformen, die eine einmal tägliche Verabreichung erlauben. Solche Darreichungsformen, die den Wirkstoff retardiert freisetzen, sind aber wegen der relativ hohen Menge an Opioid für den Missbraucher besonders attraktiv, um die gewünschten, rauschartigen, euphorisierenden Zustände möglichst rasch herbei zu führen.

Da aber retardierte Darreichungsformen, die Opioide mit Missbrauchspotential enthalten, üblicherweise selbst bei einer oralen Einnahme von mißbräuchlich hohen Mengen nicht zu dem vom Missbraucher gewünschten Kick führen, werden diese beispielsweise in Form von Tabletten oder Kapseln vorliegenden Darreichungsformen, für den Missbrauch vom Missbraucher zerkleinert, z. B. gemörsert, und geschnupft oder die Opioide aus dem so erhaltenen Pulver mit Hilfe einer wäßrigen Flüssigkeit extrahiert und die resultierende Lösung, ggf. nach Filtration durch Watte oder Zellstoff, parenteral, insbesondere intravenös, appliziert. Bei dieser Art der Verabreichung kommt es zu einem gegenüber dem oralen aber auch nasalen Missbrauch noch zusätzlich beschleunigten Anfluten des Opioides mit dem vom Missbraucher gewünschten Ergebnis, nämlich den Kick.

Zur Verhinderung von Missbrauch wurde in dem US-A- 4,070,494 vorgeschlagen, der Darreichungsform ein quellbares Mittel zuzusetzen. Dieses quillt bei der Zugabe von Wasser zur Extraktion des Opioids auf und bewirkt, dass das vom Gel separierte Filtrat nur eine geringe Menge an Wirkstoff enthält.

Ein entsprechender Ansatz zur Verhinderung eines parenteralen Missbrauchs liegt auch der in der WO 95/20947 offenbarten Mehrschichttablette zugrunde, die ein Opioid und mindestens einen Gelbildner jeweils in unterschiedlichen Schichten getrennt aufweist.

Ein weiterer Ansatz zur Verhinderung des parenteralen Missbrauchs wird in der WO 03/015531 A2 offenbart. Dort wird eine Darreichungsform enthaltend ein analgetisches Opioid und einen Farbstoff als aversives Mittel beschrieben. Die Farbe, die durch unzulässige Manipulation der Darreichungsform freigesetzt wird, soll der Missbraucher davon abhalten, diese manipulierte Darreichungsform zu verwenden. In US 2003/068392 wird der Zusatz von reizenden Substanzen zur Missbrauchsverhinderung offenbart.

Eine weitere bekannte Möglichkeit zur Erschwerung des Missbrauchs besteht darin, der Darreichungsform einen Antagonisten des Opioids, wie z. B. Naloxon oder Naltexon, oder Verbindungen, die zu physiologischen Abwehrreaktionen führen, wie z. B. Raolix Ipecacuama = Brechwurz, oder Bitterstoffe der Darreichungsform zuzusetzen.

Da aber nach wie vor in den meisten Fällen für den Missbrauch von Darreichungsformen mit kontrollierter Freisetzung eines Opioids eine Pulverisierung der Darreichungsformen notwendig ist, war es Aufgabe der vorliegenden Erfindung, die dem Missbrauch vorangehende Pulverisierung der Darreichungsform mit den einem potentiellen Missbraucher üblicherweise zur Verfügung stehenden Mitteln zu erschweren bzw. zu verhindern und somit eine Darreichungsform mit kontrollierter Freisetzung für Opioide mit Missbrauchspotential zur Verfügung zu stellen, die bei bestimmungsgemäßer Applikation die gewünschte therapeutische Wirkung bei einer einmal täglichen Verabreichung gewährleistet, aus der aber die Opioide nicht durch einfaches Pulverisieren in eine zum Missbrauch geeignete Form übergeführt werden können.

Diese Aufgabe wurde durch die Bereitstellung der erfindungsgemäßen, gegen Missbrauch gesicherten, oralen Darreichungsform (d.h. Tablette) mit kontrollierter Freisetzung wenigstens eines Opioides für eine einmal tägliche Verabreichung und zur Anwendung bei der Schmerzbekämpfung über eine Dauer von mindestens 24 Stunden gelöst, die neben wenigstens einem Opioid und/oder wenigstens einer seiner physiologisch verträglichen Verbindungen, vorzugsweise dessen Salze oder Solvate oder Derivate, vorzugsweise Amide, Ester oder Ether und/oder wenigstens eine entsprechende stereoisomere Verbindung, vorzugsweise entsprechende Enantiomeren, Stereoisomeren, Diastereoisomren oder Racemate und/oder deren physiologisch verträgliche Verbindungen wie Salze oder Solvate oder Derivate wie Amide, Ether oder Ester, mit Missbrauchspotential (A), mindestens ein synthetisches und/oder natürliches Polymer (C), ggf. wenigstens ein retardierendes Matrix-Material, ggf. mindestens einen retardierenden Überzug, ggf. physiologisch verträgliche Hilfsstoffe (B), gegebenenfalls wenigstens ein Wachs (D) umfasst, wobei die Komponente (C) bzw. (D) jeweils eine Bruchfestigkeit von mindestens 500 N, mindestens 750 N, aufweist.

Durch den Einsatz von Komponenten (C) und ggf. (D) mit der angegebenen Mindestbruchfestigkeit (gemessen wie in Anspruch 1 des Patents offenbart), in solchen Mengen, dass auch die Darreichungsform eine solche Mindestbruchfestigkeit von mindestens 500 N, vorzugsweise mindestens 750 N aufweist, gelingt es, ein Pulverisieren der Darreichungsform mit üblichen Mitteln zu verhindern und damit den anschließenden Missbrauch, vorzugsweise einen nasalen oder parenteralen Missbrauch, erheblich zu erschweren bzw. zu verhindern.

Ohne ausreichende Zerkleinerung der Darreichungsform ist eine gefahrlose parenterale, insbesondere intravenöse oder eine nasale Applikation nicht möglich oder die Extraktion des Wirkstoffs dauert dem Missbraucher zu lange oder ein Kick erfolgt bei missbräuchlicher, oraler Einnahme nicht oder nicht in ausreichender Weise, da keine spontane Freisetzung passiert.

Unter einer Zerkleinerung wird erfindungsgemäß die Pulverisierung der Darreichungsform mit üblichen Mitteln verstanden, die einem Missbraucher üblicherweise zur Verfügung stehen, wie z. B. ein Mörser und Pistill, ein Hammer, ein Schlegel oder andere gebräuchliche Mittel zum Pulverisieren unter Krafteinwirkung.

Die erfindungsgemäße Darreichungsform ist daher zur Verhinderung des parenteralen, nasalen und/oder oralen Missbrauchs von Opioiden mit Missbrauchspotential geeignet.

Opioide mit Missbrauchspotential sind dem Fachmann ebenso wie deren Dosierung und Verfahren zu deren Herstellung bekannt und können als solche, in Form ihrer entsprechenden Derivate, insbesondere Amide, Ester oder Ether, oder jeweils in Form entsprechender physiologisch verträglicher Verbindungen, insbesondere in Form ihrer Salze oder Solvate, als Racemate oder Stereoisomere in der erfindungsgemäßen Darreichungsform vorliegen. Die erfindungsgemäße Darreichungsform eignet sich auch für die Verabreichung von mehreren Opioiden. Vorzugsweise wird sie zur Verabreichung an Menschen oder Säugetieren, vorzugsweise an Menschen zur Schmerzbekämpfung über eine Dauer von mindestens 24 Stunden mit einem bestimmten Opioid eingesetzt.

Substanzen, die in die Klasse der Opioide fallen sind dem Fachmann beispielsweise aus "Opioid Analgesics" von Alan F. Casy u. a. Auflage von 1986, inbesondere auch Seite 508 bis 518, Plenum Publishing Corporation, "Analgesics" von H. Buschmann, Auflage von 2002, Seite 171 bis 245, WILEY-VCH und "Ullmann's Encyclopedia of Inustrial Chemistry" von Elmar Fiedrichs und andere, 6. Auflage, Seite 1 bis 53, WILEY-VCH bekannt. Die dort aufgeführten Opioide und deren Metabolite sind besonders bevorzugt.

Ganz besonders eignen sich die erfindungsgemäßen Darreichungsformen zur Verhinderung des Missbrauchs eines Opioids, das ausgewählt ist aus der Gruppe umfassend N-{1-[2-(4-Ethyl-5-oxo-2-tetrazolin-1-yl)ethyl]-4-methoxymethyl-4-piperidyl}propionanilid (Alfentanil), Allylprodin, Alphaprodin, Anileridin, Bemidon, Benzylmorphin, Bezitramid, 17-Cyclopropylmethyl-4,5α-epopxy-7α[(S)-1-hydroxy-1,2,2-trimethyl-propyl]-6-methoxy-6,14-*endo*-ethanomorphinan-3-ol (Buprenorphin), Butorphanol, Carfentanil, Clofedanol Clonitazen, (-)-Methyl-[3β-benzoyloxy-2β(1αH,5α*H*)-tropancarboxylat] (Cocain), 4,5α-Epoxy-3-methoxy-17-methyl-7-morphinen-6α-ol (Codein), Desomorphin, Dextromoramid, (+)-(1-Benzyl-3-dimethylamino-2-methyl-1-phenylpropyl)propionat (Dextropropoxyphen), Dezocin, Diampromid, Diamorphon, 4,5α-Epoxy-3-methoxy-17-methyl-6α-morphinanol (Dihydrocodein), 4,5α-Epoxy-17-methyl-3,6a-morphinandiol (Dihydromorphin), Dimenoxadol, Dimephetamol, Dimethylthiambuten, Dioxaphetylbutyrat, Dipipanon, Dihydromorphon, Eptazocin, Ethoheptazin, Ethylmethylthiambuten, 4,5α-Epoxy-3-ethoxy-17-methyl-7-morphinen-6α-ol (Ethylmorphin), Etonitazen, 4,5α-Epoxy-7α-(1-hydroxy-1-methylbutyl)-6-methoxy-17-methyl-6,14-*endo*-etheno-morphinan-3-ol (Etorphin), Fenpipramid, *N*-(1-Phenethyl-4-piperidyl)propionanilid (Fentanyl), Heroin, 4,5α-Epoxy-3-methoxy-17-methyl-6-morphinanon (Hydrocodon), 4,5α-Epoxy-3-hydroxy-17-methyl-6-morphinanon (Hydromorphon), Hydroxypethidin, Isomethadon, Hydroxymethylmorphinan, 1-[4-(3-Hydroxyphenyl)-1-methyl-4-piperidyl]-1-propanon (Ketobemidon), (3S,6S)-6-Dimethylamino-4,4-diphenylheptan-3-ylacetat (Levacetylmethadol), (-)-6-Dimethylamino-4,4-diphenyl-3-heptanon (Levomethadon), (-)-17-Methyl-3-morphinanol (Levorphanol), Levophenacylmorphan, Levoxemacin, Lofentanil, Meperidin, 2-Methyl-2-propyltrimethylendicarbamat, Meptazinol, Metazocin, Methadon, Methylmorphin, Metapon, 3-Methylfentanyl, 4-Methyl-fentanyl, 4,5α-Epoxy-17-methyl-7-morphinen-3,6α-diol (Morphin), Morphin-6-Glucoronid, Myrophin, Nalbuphen, Nalorphin, Narcein, Nicomorphin, 6-Dimethylamino-4,4-diphenyl-3-hexanon (Normethadon), Normorphin, Norpipanon, der geronnene Saft der zur Art Papaver somniferum gehörenden Pflanzen (Opium), 4,5α-Epoxy-14-hydroxy-3-methoxy-17-methyl-6-morphinanon (Oxycodon), Oxymorphon, Pflanzen und Pflanzenteile der zur Art Papaver somniferum (einschließlich der Unterart setigerum) gehörenden Pflanzen (Papaver somniferum), Papaveretum, 1,2,3,4,5,6-Hexahydro-6,11-dimethyl-3-(3-methyl-2-butenyl)-2,6-methano-3-benzazocin-8-ol (Pentazocin), Ethyl-(1-methyl-4-phenyl-4-piperidincarboxylat) (Pethidin), Phenadoxon, Phenomorphan, Phenazocin, Phenoperidin, Piminodin, Pholcodein, 1'-(3-Cyan-3,3-diphenylpropyl)[1,4'-bipiperidin]-4'-carboxamid (Piritramid), Proheptazin, Promedol, Properidin, Propoxyphen, Methyl{3-[4-methoxycarbonyl-4-(*N-*phenylpropanamido)piperidino]propanoat} (Remifentanil), *N*-{4-Methoxymethyl-1-[2-(2-thienyl)ethyl]-4-piperidyl}propionanilid (Sufentanil), Ethyl-(2-dimethylamino-1-phenyl-3-cyclohexen-1-carboxylat (Tilidin, cis und trans), Thebain, Tramadol, (1 R, 2R, 4S)-2-[(Dimethylamino)methyl-4-(p-fluorbenzyloxy)-1-(m-methoxyphenyl)cyclohexanol, (1R, 2R)-3-(2-Dimethylaminomethyl-cyclohexyl)-phenol, (1 S, 2S)-3(3-Dimethylamino-1-ethyl-2-methyl-propyl)-phenol, (2R, 3R)-1-Dimethylamino-3(3-methoxy-phenyl)-2-methyl-pentan-3-ol, (1 RS, 3RS, 6RS)-6-Dimethylaminomethyl-1-(3-methoxy-phenyl)-cyclohexan-1,3-diol, vorzugsweise als Racemat, 3-(2-Dimethylaminomethyl-1-hydroxy-cyclohexyl)-phenyl 2-(4-isobutylphenyl)-propionat, 3-(2-Dimethylaminomethyl-1-hydroxy-cyclohexyl)phenyl 2-(6-methoxy-naphthalen-2-yl)-propionat, 3-(2-Dimethylaminomethyl-cyclohex-1-enyl)-phenyl 2-(4-isobutyl-phenyl)-propionat, 3-(2-Dimethylaminomethyl-cyclohex-1-enyl)-phenyl 2-(6-methoxy-naphthalen-2-yl)-propionat, (RR-SS)-2-Acetoxy-4-trifluoromethyl-benzoesäure 3-(2-dimethylaminomethyl-1-hydroxy-cyclohexyl)-phenyl ester, (RR-SS)-2-Hydroxy-4-trifluoromethyl-benzoesäure 3-(2-dimethylaminomethyl-1-hydroxy-cyclohexyl)-phenyl ester, (RR-SS)-4-Chloro-2-hydroxy-benzoesäure 3-(2-dimethylaminomethyl-1-hydroxy-cyclohexyl)-phenyl ester, (RR-SS)-2-Hydroxy-4-methyl-benzoesäure 3-(2-dimethylaminomethyl-1-hydroxy-cyclohexyl)-phenyl ester, (RR-SS)-2-Hydroxy-4-methoxy-benzoesäure 3-(2-dimethylaminomethyl-1-hydroxy-cyclohexyl)-phenyl-ester, (RR-SS)-2-Hydroxy-5-nitro-benzoesäure 3-(2-dimethylaminomethyl-1-hydroxy-cyclohexyl)-phenyl ester, (RR-SS)-2',4'-Difluoro-3-hydroxy-biphenyl-4-carbonsäure 3-(2-dimethylaminomethyl-1-hydroxy-cyclohexyl)-phenyl ester sowie für entsprechende stereoisomere Verbindungen, jeweils deren entsprechende Derivate, insbesondere Amide, Ester oder Ether, und jeweils deren physiologisch verträgliche Verbindungen, insbesondere deren Salze und Solvate, besonders bevorzugt Hydrochloride, Sulfate, Saccharinate, wirksame Metabolite, Diphenoxylate, Levomethadone, Nortilidin, Piritramide und Viminol.

Die erfindungsgemäße Darreichungsform eignet sich insbesonders zur Verhinderung des Missbrauchs eines opioiden Wirkstoffes ausgewählt aus der Gruppe umfassend Oxycodon, Hydromorphon, Morphin, Oxymorphon, Tramadol und deren physiologisch verträgliche Derivate oder Verbindungen, vorzugsweise deren Salze und Solvate, vorzugsweise deren Hydrochloride, Sulfate, Saccharinate, und/oder deren Stereoisomeren bzw. entsprechenden Verbindungen und/oder Derivate.

Weiterhin eignet sich die erfindungsgemäße Darreichungsform insbesonders zur Verhinderung des Missbrauchs eines opioiden Wirkstoffes ausgewählt aus der Gruppe umfassend (2R, 3R)-1-Dimethylamino-3-(3-methoxy-phenyl)-2-methyl-pentan-3-ol, (1RS, 3RS, 6RS)-6-Dimethylaminomethyl-1-(3-methoxy-phenyl)-cyclohexane-1,3-diol, (1 R, 2R)-3-(2-Dimethylaminomethyl-cyclohexyl)-phenol, deren physiologisch verträglichen Salze, vorzugsweise Hydrochloride, Sulfate, Saccharinate, physiologisch verträgliche Enantiomeren, Stereoisomeren, Diastereomeren und Racemate und deren physiologisch verträglichen Derivate, vorzugsweise Ether, Ester oder Amide.

Diese Verbindungen bzw. deren Herstellungsverfahren sind in der EP-A-693475 bzw. EP-A-780369 beschrieben.

Die Dosierung in der retardierten Darreichungsform wird so gewählt, dass sie für eine einmal tägliche Verabreichung gewährleistet ist. Die entsprechenden Dosierungen sind dem Fachmann bekannt.

In der erfindungsgemäßen Darreichungsform liegt der Gehalt des Wirkstoffes vorzugsweise zwischen 0,05 und 80 Gew.%, besonders bevorzugt zwischen 0,05 und 60 Gew.% und ganz besonders bevorzugt zwischen 0,05 und 40 Gew.%.

Zur Erzielung der notwendigen Bruchfestigkeit der erfindungsgemäßen Darreichungsform werden mindestens ein synthetisches, halbsynthetisches und/oder natürliches Polymer (C) mit einer Bruchfestigkeit, gemessen nach der in Anspruch 1 des Patents offenbarten Methode, von mindestens 500 N, vorzugsweise 750 N, eingesetzt. Bevorzugt wird hierfür mindestens ein Polymeres ausgewählt aus der Gruppe umfassend Polyalkylenoxide, vorzugsweise Polymethylenoxide, Polyethylenoxide, Polypropylenoxide, Polyolefine, vorzugsweise Polyethylene, Polypropylene, Polyvinylchloride, Polycarbonate, Polystyrole, Poly(meth)acrylate, deren Copolymerisate und Mischungen aus mindestens zwei Vertretern der genannten Polymerklassen oder Polymeren eingesetzt. Besonders bevorzugt wird ein wasserlösliches oder wasserquellbares Polymer eingesetzt. Bevorzugt sind hochmolekulare, thermoplastische Polyalkylenoxide. Besonders bevorzugt sind Polyethylenoxide mit einem Molekulargewicht von mindestens 0,5 Mio., vorzugsweise von mindestens 1 Mio., besonders bevorzugt von 1 Mio. bis 15 Mio., bestimmt durch rheologische Messungen. Diese Polyethylenoxide weisen eine Viskosität bei 25 °C von 4500 bis 17600 cP, gemessen an einer 5 Gew.% wässrigen Lösung des Polymeren mit Hilfe eines Brookfield Viskosimeters, Model RVF (Spindel Nr. 2 / Rotationsgeschwindigkeit 2 rpm), von 400 bis 4000 cP, gemessen an einer 2 Gew.% wässrigen Lösung des Polymeren mit Hilfe des genannten Viskosimeters (aber mit Spindel Nr. 1 bzw. 3 / Rotationsgeschwindigkeit 10 rpm) bzw. von 1650 bis 10000 cP, gemessen an einer 1 Gew.% wässrigen Lösung des Polymers mit Hilfe des genannten Viskosimeters (aber mit Spindel Nr. 2 /Rotationsgeschwindigkeit 2 rpm) auf (vgl. Handbook of Pharmaceutical Excipients von Raymond C. Rowe u. a., Ausgabe 4., 2003, Seite 460).

Die Polymeren werden vorzugsweise als Pulver zur Herstellung der erfindungsgemäßen Darreichungsform eingesetzt. Sie können wasserlöslich oder wasserquellbar sein.

Vorzugsweise wird die Komponente (C) in einer Menge von 20 bis 99,9 Gew.%, besonders bevorzugt von wenigstens 35 Gew.%, ganz besonders bevorzugt von wenigstens 50 Gew.%, bezogen auf das Gesamtgewicht der Darreichungsform, eingesetzt.

Als Hilfsstoffe (B) können die üblichen für die Formulierung von festen Darreichungsformen bekannte Hilfsstoffe verwendet werden. Vorzugsweise sind diese Weichmacher, wie Polyethylenglykol, in Mengen von 0,01 bis 20 Gew.%, besonders bevorzugt bis 15 Gew.% und ganz besonders bevorzugt bis 10 Gew.%, Hilfsstoffe, die die Wirkstofffreisetzung beeinflussen, wie nachstehend aufgeführt vorzugsweise hydrophobe oder hydrophile, vorzugsweise hydrophile Polymere, ganz besonders bevorzugt Hydroxypropylmethylcellulose oder Hydroxypropylcellulose, und/oder Antioxidantien. Als Antioxidantien eignen sich Ascorbinsäure, Butylhydroxyanisol, Butylhydroxytoluol, Salze der Ascorbinsäure, Monothioglyzerin, phosphorige Säure, Vitamin C, Vitamin E und dessen Derivate, Natriumbisulfit, besonders bevorzugt Butylhydroxytoluol (BHT) oder Butylhydroxyanisol (BHA) und α-Tocopherol.

Das Antioxidanz wird vorzugsweise in Mengen von 0,01 bis 10 Gew.%, vorzugsweise 0,03 bis 5 Gew.%, bezogen auf das Gesamtgewicht der Darreichungsform, eingesetzt.

Des weiteren können neben den vorstehend genannten Polymeren zusätzlich zur Erzielung der notwendigen Bruchfestigkeit der erfindungsgemäßen Darreichungsform mindestens ein natürliches, halbsynthetisches oder synthetisches Wachs (D) mit einer Bruchfestigkeit, gemessen nach der in Anspruch 1 des Patents offenbarten Methode von mindestens 500 N, vorzugsweise 750 N, eingesetzt werden. Bevorzugt sind Wachse mit einem Erweichungspunkt von mindestens 60°C. Besonders bevorzugt sind Carnaubawachs und Bienenwachs. Ganz besonders bevorzugt ist Carnaubawachs. Carnaubawachs ist ein natürliches Wachs, das aus den Blättern der Carnaubapalme gewonnen wird und einen Erweichungspunkt von höchstens 90°C aufweist. Bei einem zusätzlichen Einsatz der Wachskomponente wird diese zusammen mit wenigstens einem Polymeren (C), vorzugsweise mit wenigstens einem Polyethylenoxid, in solchen Mengen eingesetzt, dass die Darreichungsform eine Bruchfestigkeit von mindestens 500 N, vorzugsweise mindestens 750 N, gemessen nach der in Anspruch 1 des Patents angegebenen Methode, aufweist.

Die erfindungsgemäßen Darreichungsformen zeichnen sich dadurch aus, dass sie aufgrund ihrer Härte nicht mit Hilfe von üblichen Zerkleinerungsmitteln, wie Mörser und Pistill zu pulverisieren sind. Ein oraler, parenteraler, insbesondere intravenöser oder nasaler Missbrauch ist dadurch praktisch ausgeschlossen. Um jedoch jeden möglichen Missbrauch der erfindungsgemäßen Darreichungsformen vorzubeugen, können die erfindungsgemäßen Darreichungsformen in einer bevorzugten Ausführungsform als Hilfsstoffe (B) weitere missbrauchserschwerende bzw. - verhindernde Mittel enthalten.

So kann die erfindungsgemäße, gegen Missbrauch gesicherte Darreichungsform, die neben wenigstens einem Opioid mit Missbrauchspotential mindestens einem Polymer (C) und ggf. mindestens einem Wachs (D) noch wenigstens eine der nachfolgenden missbrauchsverhindernden Komponenten (a)-(f) als Hilfsstoffe (B) aufweisen:
(a) wenigstens einen den Nasen- und/oder Rachenraum reizenden Stoff,
(b) wenigstens ein viskositätserhöhendes Mittel, das in einem mit Hilfe einer notwendigen Mindestmenge an einer wässrigen Flüssigkeit, vorzugsweise als ein aus der Darreichungsform gewonnenes wässriges Extrakt, ein Gel bildet, welches vorzugsweise beim Einbringen in eine weitere Menge einer wässrigen Flüssigkeit visuell unterscheidbar bleibt,
(c) weinigstens einen Antagonisten für die vorhandenen Opioide mit Missbrauchspotential,
(d) wenigstens ein Emetikum,
(e) wenigstens einen Farbstoff als aversives Mittel,
(f) wenigstens einen Bitterstoff.

Die Komponenten (a) bis (f) sind jede für sich allein als zusätzliche Sicherung der erfindungsgemäßen Darreichungsform gegen Missbrauch geeignet. So eignet sich die Komponente (a) bevorzugt zur Sicherung gegen nasalen, oralen und/oder parenteralen, vorzugsweise intravenösen, Missbrauch, die Komponente (b) bevorzugt gegen parenteralen, besonders bevorzugt intravenösen und/oder nasalen Missbrauch, die Komponente (c) bevorzugt gegen nasalen und/oder parenteralen, besonders bevorzugt intravenösen Missbrauch, die Komponente (d) vorzugsweise gegen parenteralen, besonders bevorzugt intravenösen, und/oder oralen und/oder nasalen Missbrauch, die Komponente (e) als visuelles Abschreckungsmittel gegen oralen oder parenteralen Missbrauch und die Komponente (f) gegen oralen oder nasalen Missbrauch. Durch die Mitverwendung von wenigstens einer der vorstehend genannten Komponenten gelingt es, bei den erfindungsgemäßen Darreichungsformen noch effektiver den Missbrauch zu erschweren.

In einer Ausführungsform kann die erfindungsgemäße Darreichungsform auch zwei oder mehrere der Komponenten (a)-(f) in einer Kombination aufweisen, vorzugsweise in den Kombinationen (a), (b) und ggf. (c) und/oder (f) und/oder (e) bzw. (a), (b) und ggf. (d) und/oder (f) und/oder (e).

In einer weiteren Ausführungsform kann die erfindungsgemäße Darreichungsform sämtliche Komponenten (a)-(f) aufweisen.

Sofern die erfindungsgemäße Darreichungsform als zusätzlichen Schutz gegen Missbrauch die Komponente (a) umfaßt, kommen als den Nasen- und/oder Rachenraum reizende Stoffe erfindungsgemäß sämtliche Stoffe in Betracht, die bei entsprechender Applikation über den Nasen- und/oder Rachenraum eine Reaktion des Körpers hervorrufen, die entweder für den Missbraucher so unangenehm ist, dass der die Applikation nicht weiter fortsetzen will oder kann, z. B. ein Brennen, oder die auf physiologische Art und Weise einer Aufnahme der (des) entsprechenden Opioide(s) und/oder Opiate(s) entgegenwirken, z. B. über eine vermehrte nasale Sekretbildung oder Niesen. Diese üblicherweise den Nasen- und/oder Rachenraum reizenden Stoffe können auch bei parenteraler, insbesondere intravenöser, Applikation ein sehr unangenehmes Gefühl bis hin zu unerträglichen Schmerzen verursachen, so dass der Missbraucher die Applikation nicht länger fortsetzen will oder kann.

Besonders geeignete, den Nasen- und/oder Rachenraum reizende Stoffe sind solche Stoffe, die ein Brennen, einen Juckreiz, einen Niesreiz, eine vermehrte Sekretbildung oder eine Kombination mindestens zweier dieser Reize verursachen. Entsprechende Stoffe und deren üblicherweise einzusetzenden Mengen sind dem Fachmann an sich bekannt oder können durch einfache Vorversuche ermittelt werden.

Der den Nasen- und/oder Rachenraum reizende Stoff der Komponente (a) basiert vorzugsweise auf einem oder mehreren Inhaltsstoffen oder einem oder mehreren Pflanzenteilen wenigstens einer Scharfstoffdroge.

Entsprechende Scharfstoffdrogen sind dem Fachmann an sich bekannt und werden beispielsweise in "Pharmazeutische Biologie - Drogen und ihre Inhaltsstoffe" von Prof. Dr. Hildebert Wagner, 2., bearbeitete Auflage, Gustav Fischer Verlag, Stuttgart-New York, 1982, Seiten 82 ff., beschrieben.

Unter Darreichungseinheit wird eine separate bzw. separierbare Dosiseinheit, wie z. B. eine Tablette oder eine Kapsel, verstanden.

Vorzugsweise kann der erfindungsgemäßen Darreichungsform als Komponente (a) einer oder mehrere Inhaltsstoffe wenigstens einer Scharfstoffdroge, ausgewählt aus der Gruppe bestehend aus Allii sativi Bulbus, Asari Rhizoma c. Herba, Calami Rhizoma, Capsici Fructus (Paprika), Capsici Fructus acer (Cayennepfeffer), Curcumae longae Rhizoma, Curcumae xanthorrhizae Rhizoma, Galangae Rhizoma, Myristicae Semen, Piperis nigri Fructus (Pfeffer), Sinapis albae (Erucae) Semen, Sinapis nigri Semen, Zedoariae Rhizoma und Zingiberis Rhizoma, besonders bevorzugt aus der Gruppe bestehend aus Capsici Fructus (Paprika), Capsici Fructus acer (Cayennepfeffer) und Piperis nigri Fructus (Pfeffer), hinzugefügt werden.

Bei den Inhaltsstoffen der Scharfstoffdrogen handelt es sich bevorzugt um o-Methoxy(Methyl)-phenol-Verbindungen, Säureamid-Verbindungen, Senföle oder Sulfidverbindungen oder um davon abgeleiteten Verbindungen.

Besonders bevorzugt ist wenigstens ein Inhaltsstoff der Scharfstoffdrogen ausgewählt aus der Gruppe bestehend aus Myristicin, Elemicin, Isoeugenol, α-Asaron, Safrol, Gingerolen, Xanthorrhizol, Capsaicinoiden, vorzugsweise Capsaicin, Capsaicin- Derivate, wie N-vanillyl -9E-octadecenamid, Dihydrocapsaicin, Nordihydrocapsaicin, Homocapsaicin, Norcapsaicin, und Nomorcapsaicin, Piperin, vorzugsweise trans-Piperin, Glucosinolaten, vorzugsweise auf Basis von nichtflüchtigen Senfölen, besonders bevorzugt auf Basis von p-Hydroxybenzylsenföl, Methylmercaptosenföl oder Methylsulfonylsenföl, und von diesen Inhaltsstoffen abgeleiteten Verbindungen.

Vorzugsweise kann die erfindungsgemäße Darreichungsform die Pflanzenteile der entsprechenden Scharfstoffdrogen in einer Menge von 0,01 bis 30 Gew.-%, besonders bevorzugt 0,1 bis 0,5 Gew.-%, jeweils bezogen auf das Gesamtgewicht der Darreichungseinheit, enthalten.

Kommen ein oder mehrere Inhaltsstoffe entsprechender Scharfstoffdrogen zum Einsatz, beträgt deren Menge in einer erfindungsgemäßen Darreichungseinheit bevorzugt 0,001 bis 0,005 Gew.-%, bezogen auf das Gesamtgewicht der Darreichungseinheit.

Eine weitere Möglichkeit, bei der erfindungsgemäßen Darreichungsform zusätzlich gegen Missbrauch vorzubeugen, besteht darin, wenigstens ein viskositätserhöhendes Mittel als weitere Missbrauchs-verhindernde Komponente (b) der Darreichungsform zuzusetzen, die in einer notwendigen Mindestmenge an einer wässrigen Flüssigkeit, vorzugsweise als ein aus der Darreichungsform gewonnenes wässriges Extrakt, ein Gel bildet, das kaum gefahrlos applizierbar ist und vorzugsweise auch beim Einbringen in eine weitere Menge einer wässrigen Flüssigkeit visuell unterscheidbar bleibt.

Visuelle Unterscheidbarkeit im Sinne der vorliegenden Anmeldung bedeutet, dass das mit Hilfe einer notwendigen Mindestmenge an wässriger Flüssigkeit gebildete, Opioid- und/oder Opiat-haltige Gel beim Einbringen, vorzugsweise mit Hilfe einer Injektionsnadel, in eine weitere Menge wäßriger Flüssigkeit von 37°C im wesentlichen unlöslich und zusammenhängend bleibt und nicht auf einfache Weise so dispergiert werden kann, dass eine parenterale, insbesondere intravenöse, gefahrlose Applikation möglich ist. Vorzugsweise beträgt die Dauer der visuellen Unterscheidbarkeit wenigstens eine Minute, vorzugsweise mindestens 10 Minuten.

Die Viskositätserhöhung zum Gel führt dazu, dass dessen Nadelgängigkeit bzw. Spritzbarkeit erschwert oder sogar unmöglich gemacht wird. Sofern das Gel visuell unterscheidbar bleibt, bedeutet dies, dass das erhaltene Gel beim Einbringen in eine weitere Menge wäßriger Flüssigkeit, z. B. durch Einspritzen in Blut, zunächst in Form eines weitgehend zusammenhängenden Fadens erhalten bleibt, der zwar durch mechanische Einwirkung in kleinere Bruchstücke zerteilt, nicht aber so dispergiert oder sogar gelöst werden kann, dass eine parenterale, insbesondere intravenöse, Applikation gefahrlos möglich ist. In Kombination mit mindestens einer weiteren vorhandenen Komponente (a), (d) bis (f) führt dies zusätzlich zu unangenehmem Brennen, Erbrechen, schlechtem Geschmack und/oder zur visuellen Abschreckung.

Eine intravenöse Applikation eines entsprechenden Gels würde daher mit großer Wahrscheinlichkeit zur Verstopfung von Gefäßen, verbunden mit schweren gesundheitlichen Schaden des Missbrauchers führen.

Zur Überprüfung, ob ein viskositätserhöhendes Mittel als Komponente (b) zur Anwendung in der erfindungsgemäßen Darreichungsform geeignet ist, wird (werden) das (die) Opioid(e) und/oder Opiat(e) mit dem viskositätserhöhenden Mittel gemischt und in 10 ml Wasser bei einer Temperatur von 25 °C suspendiert. Bildet sich hierbei ein Gel, welches den obenstehend genannten Bedingungen genügt, eignet sich das entsprechende viskositätserhöhende Mittel zur zusätzlichen Missbrauchs-Vorbeugung bzw. - Verhinderung bei den erfindungsgemäßen Darreichungsformen.

Sofern die durch das erfindungsgemäße Verfahren erhaltene Darreichungsform die Komponente (b) hinzugefügt wird, kommen vorzugsweise eine oder mehrere viskositätserhöhende Mittel zum Einsatz, die ausgewählt sind aus der Gruppe umfassend mikrokristalliner Cellulose mit 11 Gew.-% Carboxymethylcellulose-Natrium (Avicel^{®} RC 591), Carboxymethylcellulose-Natrium (Blanose^{®}, CMC-Na C300P^{®}, Frimulsion BLC-5^{®}, Tylose C300 P^{®}), Polyacrylsäure (Carbopol^{®} 980 NF, Carbopol^{®} 981), Johannisbrotkernmehl (Cesagum^{®} LA-200, Cesagum^{®} LID/150, Cesagum^{®} LN-1), Pektine, vorzugsweise aus Citrusfrüchten oder Äpfeln (Cesapectin^{®} HM Medium Rapid Set), Wachsmaisstärke (C*Gel 04201^{®}), Natriumalginat (Frimulsion ALG (E401+)^{®}), Guarkernmehl (Frimulsion BM^{®}, Polygum 26/1-75^{®}), lota-Carrageen (Frimulsion D021^{®}), Karaya Gummi, Gellangummi (Kelcogel F^{®}, Kelcogel LT100^{®}), Galaktomannan (Meyprogat 150^{®}), Tarakernmehl (Polygum 43/1^{®}), Propylenglykoalginat (Protanal-Ester SD-LB^{®}), Natrium-Hyaluronat, Tragant, Taragummi (Vidogum SP 200^{®}), fermentiertes Polysaccharid- Welan Gum (K1A96), Xanthane wie Xanthan-Gummi (Xantural 180^{®}). Xanthane sind besonders bevorzugt. Die in Klammern angegebenen Bezeichnungen sind die Handelsnamen, unter denen die jeweiligen Materialien am Markt geführt sind. Im allgemeinen ist eine Menge von 0,1 bis 20 Gew.% besonders bevorzugt 0,1 bis 15 Gew.%, bezogen auf die Gesamtmenge der Darreichungsform, der/des genannten viskositätserhöhenden Mittels ausreichend, um die vorstehend genannten Bedingungen zu erfüllen.

Die viskositätserhöhenden Mittel der Komponente (b), sofern vorgesehen, liegen in der erfindungsgemäßen Darreichungsform bevorzugt in Mengen von mindestens 5 mg pro Darreichungseinheit, d.h. pro Dosiereinheit vor.

In einer besonders bevorzugten Ausführungsform der vorliegenden Erfindung kommen als Komponente (b) solche viskositätserhöhenden Mittel zum Einsatz, die vorzugsweise durch Extraktion aus der Darreichungsform mit der notwendigen Mindestmenge an wäßriger Flüssigkeit ein Gel bilden, das Luftblasen einschließt. Die so erhaltenen Gele zeichnen sich durch ein trübes Erscheinungsbild aus, durch das der potentielle Missbraucher zusätzlich optisch gewarnt und von dessen parenteraler Applikation abgehalten wird.

Die Komponente (C) kann auch gegebenenfalls als zusätzliches viskositätserhöhendes Mittel dienen, das mit Hilfe einer notwendigen Mindestmenge an einer wäßrigen Flüssigkeit ein Gel bildet.

Es ist auch möglich, die viskositätserhöhende Komponente und die übrigen Bestandteile der erfindungsgemäßen Darreichungsform voneinander räumlich getrennt anzuordnen.

Des weiteren kann die erfindungsgemäße Darreichungsform zusätzlich zur Vorbeugung und Sicherung gegen Missbrauch die Komponente (c) aufweisen, nämlich einen oder mehrere Antagonisten für das (die) Opioid(e) und/oder Opiat(e) mit Missbrauchspotential, wobei der Antagonist vorzugsweise räumlich getrennt von den übrigen Bestandteilen der erfindungsgemäßen Darreichungsform angeordnet ist und keine Wirkung bei bestimmungsgemäßer Verwendung entfalten soll.

Geeignete Antagonisten zur Verhinderung des Missbrauchs von Opioiden sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäßen Darreichungsform vorliegen.

Als Antagonist kommt bevorzugt ein Antagonist ausgewählt aus der Gruppe umfassen Naloxon, Naltrexon, Nalmefen, Nalid und Nalmexon jeweils ggf. in Form einer entsprechenden physiologisch verträglichen Verbindung, insbesondere in Form einer Base, eines Salzes oder Solvates, zum Einsatz. Vorzugsweise werden die entsprechenden Antagonisten, sofern eine Ausrüstung mit der Komponente (c) vorgesehen ist, in einer Menge von mindestens 1 mg, besonders bevorzugt in einer Menge von 3 bis 100 mg, ganz besonders bevorzugt in einer Menge von 5 bis 50 mg pro Darreichungsform, d.h. pro Dosiereinheit eingesetzt.

Vorzugsweise weist die erfindungsgemäße Darreichungsform die Antagonistenkomponente in einer üblichen, dem Fachmann bekannten der therapeutischen Dosierung, besonders bevorzugt in einer Dosierung, die gegenüber der üblichen Dosierung verdoppelt bis verdreifacht ist.

Sofern die Kombination zur zusätzlichen Vorbeugung und Sicherung der erfindungsgemäßen Darreichungsform gegen Missbrauch die Komponente (d) umfaßt, kann sie wenigstens ein Emetikum aufweisen, das vorzugsweise in einer räumlich getrennten Anordnung von den übrigen Komponenten der erfindungsgemäßen Darreichungsform vorliegen und bei bestimmungsgemäßer Anwendung keine Wirkung im Körper entfalten sollte.

Geeignete Emetika zur zusätzlichen Verhinderung des Missbrauchs eines Opioides sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäßen Darreichungsform vorliegen.

Für die erfindungsgemäße Darreichungsform kommt bevorzugt ein Emetikum auf Basis eines oder mehrerer Inhaltsstoffe von Radix Ipecacuanhae (Brechwurzel), vorzugsweise auf Basis des Inhaltsstoffes Emetin, in Betracht, wie sie z. B. in "Pharmazeutische Biologie - Drogen und ihre Inhaltsstoffe" von Prof. Dr. Hildebert Wagner, 2., bearbeitete Auflage, Gustav Fischer Verlag, Stuttgart, New York 1982 beschrieben werden.

Vorzugsweise kann die erfindungsgemäße Darreichungsform als Komponente (d) das Emetikum Emetin aufweisen, bevorzugt in einer Menge von wenigstens 3 mg, besonders bevorzugt wenigstens 10 mg und ganz besonders bevorzugt in einer Menge von wenigstens 20 mg pro Darreichungsform, d.h. Dosiereinheit.

Ebenfalls bevorzugt kann als Emetikum Apomorphin als zusätzliche Missbrauchssicherung zum Einsatz kommen, vorzugsweise in einer Menge von vorzugsweise wenigstens 3 mg, besonders bevorzugt wenigstens 5 mg und ganz besonders bevorzugt wenigstens 7 mg pro Dosiereinheit.

Sofern die erfindungsgemäße Darreichungsform die Komponente (e) als zusätzlichen missbrauchsverhindernden Hilfsstoff enthält, so wird durch den Einsatz eines solchen Farbstoffes, insbesondere bei dem Versuch, das (die) Opioid(e) für eine parenterale, vorzugsweise intravenöse Applikation, zu extrahieren, eine intensive Farbgebung einer entsprechenden wässrigen Lösung hervorgerufen, die zur Abschreckung beim potentiellen Missbraucher führen kann. Auch ein oraler Missbrauch, der üblicherweise über eine wässrige Extraktion der (des) Opioide(s) eingeleitet wird, kann durch diese Farbgebung verhindert werden. Geeignete Farbstoffe sowie die für die notwendige Abschreckungswirkung erforderlichen Mengen sind der WO 03/015531 zu entnehmen.

Sofern die erfindungsgemäße Darreichungsform als zusätzlichen missbrauchsverhindernden Hilfsstoff die Komponente (f) enthält, so wird durch diesen Zusatz von wenigstens einem Bitterstoff durch die damit eintretende Geschmacksverschlechterung der Darreichungsform der orale und/oder nasale Missbrauch zusätzlich verhindert.

Geeignete Bitterstoffe sowie die für den Einsatz wirksamen Mengen sind der US-2003/0064099 zu entnehmen, deren entsprechende Offenbarung als Offenbarung der vorliegenden Anmeldung gelten soll und hiermit als Referenz eingeführt wird. Vorzugsweise eignen sich als Bitterstoffe Aromaöle, vorzugsweise Pfefferminzöl, Eukalyptusöl, Bittermandelöl, Menthol, Fruchtaromastoffe, vorzugsweise Aromastoffe von Zitronen, Orangen, Limonen, Grapefruit oder Mischungen davon, und/oder Denatonium-Benzoat (Bitrex ^{®}). Besonders bevorzugt wird Denatonium-Benzoat eingesetzt.

Zur Gewährleistung einer einmal täglichen Verabreichung weist die erfindungsgemäße Darreichungsform das (die) Opioid(e) und/oder Opiat(e) mit Missbrauchspotential wenigstens zum Teil in retardierter Form auf, wobei die Retardierung der Wirkstofffreisetzung mit Hilfe von üblichen, dem Fachmann bekannten Materialien und Verfahren erzielt werden kann, beispielsweise durch Einbetten der (des) Opioide(s) in eine retardierende Matrix oder durch das Aufbringen eines oder mehrerer retardierender Überzüge. Die Opioid-Abgabe muß aber so gesteuert sein, dass die vorstehend genannten Bedingungen jeweils erfüllt sind, z. B. das bei bestimmungsgemäßer Applikation der Darreichungsform das (die) Opioid(e) praktisch komplett freigesetzt wird (werden), bevor die ggf. vorhandenen Komponente (C) und/oder (D) eine beeinträchtigende Wirkung entfalten können. Insbesondere, muß die Freisetzung des Opioids eine analgetische Wirkung über mindestens 24 Stunden gewährleisten.

Sofern die Freisetzung der (des) Opioide(s) aus der erfindungsgemäßen Darreichungsform mit Hilfe mindestens eines retardierenden Überzugs gesteuert wird, kann der retardierende Überzug aus üblichen, dem Fachmann bekannten Materialien bestehen.

In einer bevorzugten Ausführungsform der erfindungsgemäßen Darreichungsformen basiert der retardierende Überzug vorzugsweise auf einem wasserunlöslichen, gegebenenfalls modifizierten natürlichen und/oder synthetischen Polymeren oder auf einem natürlichen, halbsynthetischen oder synthetischen Wachs oder auf einem Fett oder einem Fettalkohol oder auf einem Gemisch aus wenigstens zwei der vorstehend genannten Komponenten.

Zur Herstellung eines retardierenden Überzugs werden als wasserunlösliche Polymere vorzugsweise Poly(meth)acrylate, besonders bevorzugt Poly(C₁₋₄)-alkyl(meth)acrylate, Poly(C₁₋₄)-dialkylamino-(C₁₋₄)-alkyl(meth)acrylate und/oder deren Copolymere, ganz besonders bevorzugt Copolymere aus Ethylacrylat und Methylmethacrylat mit einem molaren Verhältnis der Monomeren von 2 : 1 (Eudragit NE30D^{®}), Copolymere aus Ethylacrylat, Methylmethacrylat und Trimethylammoniumethylmethacrylat-chlorid mit einem molaren Verhältnis der Monomeren von 1 : 2 : 0,1 (Eudragit RS^{®}), Copolymere aus Ethylacrylat, Methylmethacrylat und Trimethylammoniumethylmethacrylatchlorid mit einem molaren Verhältnis der Monomeren von 1 : 2 : 0,2 (Eudragit RL^{®}) oder ein Gemisch aus wenigstens zwei dieser vorstehend genannten Copolymeren eingesetzt. Diese Überzugsmaterialien sind als 30 Gew.%-ige wäßrige Latexdispersionen, d.h. als Eudragit RS30D^{®}, Eudragit NE30D^{®} bzw. Eudragit RL30D^{®} am Markt erhältlich und werden als solche auch als Überzugsmaterial bevorzugt eingesetzt.

Ebenfalls bevorzugt können als wasserunlösliche Polymere zur Herstellung eines retardierenden Überzugs der erfindungsgemäßen Darreichungsformen Polyvinylacetate ggf. in Kombination mit weiteren Hilfsstoffen eingesetzt werden. Diese sind als wäßrige Dispersion enthaltend 27 Gew.-% Polyvinylacetat, 2,5 Gew.-% Povidon und 0,3 Gew.-% Natriumlaurylsulfat (Kollicoat SR 30 D^{®}) am Markt erhältlich.

In einer weiteren bevorzugten Ausführungsform basieren die retardierenden Überzüge der erfindungsgemäßen Darreichungsform auf wasserunlöslichen Cellulosederivaten, vorzugsweise Alkylcellulosen wie z. B. Ethylcellulose, oder Celluloseestern, wie z. B. Celluloseacetat. Die Überzüge aus Ethylcellulose oder Celluloseacetat werden bevorzugt aus wäßriger Pseudolatexdispersion aufgebracht. Wäßrige Ethylcellulose-Pseudolatexdispersionen werden als 30 Gew.%-ige Dispersionen (Aquacoat^{®}) oder als 25 Gew.%-ige Dispersionen (Surelease^{®}) am Markt geführt.

Sofern der retardierende Überzug auf einem wasserunlöslichen, gegebenenfalls modifizierten natürlichen und/oder synthetischen Polymeren basiert, kann die Überzugsdispersion oder Lösung neben dem entsprechendem Polymer einen üblichen, dem Fachmann bekannten, physiologisch verträglichen Weichmacher aufweisen, um die notwendige Mindestfilmtemperatur zu senken.

Geeignete Weichmacher sind beispielsweise lipophile Diester aus einer aliphatischen oder aromatischen Dicarbonsäure mit C₆-C₄₀ und einem aliphatischen Alkohol mit C₁-C₈, wie z. B. Dibutylphthalat, Diethylphthalat, Dibutylsebacat oder Diethylsebacat, hydrophile oder lipophile Ester der Zitronensäure, wie z. B. Triethylcitrat, Tributylcitrat, Acetyltributylcitrat oder Acetyltriethylcitrat, Polyethylenglycole, Propylenglycol, Ester des Glycerins, wie z. B. Triacetin, Myvacet^{®} (acetylierte Mono- und Diglyceride, C₂₃H₄₄O₅ bis C₂₅H₄₇O₇), mittelkettige Triglyceride (Miglyol^{®}), Ölsäure oder Gemische aus wenigstens zwei der genannten Weichmacher. Vorzugsweise enthalten wäßrige Dispersionen von Eudragit RS^{®} und gegebenenfalls Eudragit RL^{®} Triethylcitrat.

Vorzugsweise enthält ein retardierender Überzug der erfindungsgemäßen Darreichungsform Weichmacher in Mengen von 5 bis 50 Gew-%., besonders bevorzugt 10 bis 40 Gew.-% und ganz besonders bevorzugt 10 bis 30 Gew-%, bezogen auf die Menge des eingesetzten Polymers. In Einzelfällen, beispielsweise für Celluloseacetat können auch höhere Mengen an Weichmachern eingesetzt werden.

Des weiteren kann ein retardierender Überzug weitere übliche, dem Fachmann bekannte Hilfsstoffe, wie z. B. Gleitmittel, vorzugsweise Talkum oder Glycerinmonostearat, Farbpigmente, vorzugsweise Eisenoxide oder Titandioxid, oder Tenside, wie z. B. Tween 80^{®} aufweisen.

Das erhaltene Freisetzungsprofil der (des) Opioide(s) kann weiterhin durch übliche, dem Fachmann bekannten Möglichkeiten, wie z. B. durch die Dicke des Überzugs oder durch den Einsatz weiterer Hilfsstoffe als Bestandteile des Überzugs eingestellt werden. Geeignete Hilfsstoffe sind beispielsweise hydrophile oder pH-abhängige Porenbildner, wie z. B. Natrium-Carboxymethylcellulose, Celluloseacetatphthalat, Hydroxypropylmethylcelluloseacetatsuccinat, Lactose, Polyethylenglykol oder Mannitol oder wasserlösliche Polymere, wie z. B. Polyvinylpyrrolidon oder wasserlösliche Cellulosen, vorzugsweise Hydroxypropylmethylcellulose oder Hydroxypropylcellulose.

Die erfindungsgemäßen Darreichungsformen zur Freisetzung der (des) Opioide(s) können zusätzlich auch einen magensaftresistenten und/oder geschmackskaschierenden Überzug aufweisen, der sich pH-abhängig auflöst. Durch diesen Überzug kann erreicht werden, dass die erfindungsgemäßen Darreichungsformen den Magentrakt unaufgelöst passieren und das (die) Opioid(e) erst im Darmtrakt zur Freisetzung gelangt.

Der magensaftresistente Überzug basiert vorzugsweise auf Methacrylsäure/Alkylmethacrylat-Copolymeren, vorzugsweise Methylmethacrylat, wie Methacrylsäure oder Ethylenmethacrylat-Copolymeren mit einem molaren Verhältnis der jeweiligen Monomeren von 1:1 bis 1:2 wie Eudragit L^{®} oder Eudragit S^{®}, Eudragit L30D-55^{®}, Eudragit FS^{®}.

Ein Retardüberzug kann nach üblichen, dem Fachmann bekannten Verfahren, wie z. B. durch Aufsprühen von Lösungen, Dispersionen oder Suspensionen, durch Schmelzverfahren oder durch Pulverauftragsverfahren aufgebracht werden. Die Lösungen, Dispersionen oder Suspensionen können in Form von wäßrigen oder organischen Lösungen oder Dispersionen eingesetzt werden. Dabei werden wäßrige Dispersionen bevorzugt eingesetzt. Als organische Lösungsmittel können Alkohole, beispielsweise Ethanol oder Isopropanol, Ketone, wie z. B. Aceton, Ester, beispielsweise Ethylacetat, verwendet werden, wobei Alkohole und Ketone bevorzugt eingesetzt werden. Die Überzugsverfahren sind aus dem Stand der Technik, z. B. H. Sucker, Georg Thieme Verlag, 1991, Seiten 347 ff. bekannt.

Sofern die erfindungsgemäße Darreichungsform in multipartikulärer Form vorliegt, wird der retardierende Überzug vorzugsweise so aufgebracht, dass man die multipartikulären Formen enthaltend das (die) Opioid(e) nach ihrer Herstellung mit den jeweiligen Polymeren und ggf. weiteren Hilfsstoffen aus wässrigen und/oder organischen Medien, vorzugsweise aus wässrigen Medien, mit Hilfe des Wirbelschichtverfahrens überzieht und den Überzug vorzugsweise gleichzeitig bei üblichen Temperaturen in der Wirbelschicht trocknet.

Vorzugsweise erfolgt die Trocknung eines Überzuges auf Poly(meth)acrylatbasis bei Temperaturen im Bereich von 30 bis 50°C, besonders bevorzugt von 35 bis 45 °C. Für Überzüge auf Cellulosebasis, wie z. B. Ethylcellulose, erfolgt die Trocknung bevorzugt bei einer Temperatur im Bereich von 50 bis 80°C, besonders bevorzugt im Bereich von 55 bis 65 °C. Wenn notwendig, kann sich an die Trocknung noch eine Temperung anschließen, um ein stabiles Freisetzungsprofil zu erhalten.

Die retardierte Freisetzung des Wirkstoffes aus der erfindungsgemäßen Darreichungsform kann auch durch Einbettung der (des) Opioide(s) in eine retardierende Matrix erzielt werden.

Als Materialien für eine retardierende Matrix können vorzugsweise physiologisch verträgliche, hydrophile Polymere, bevorzugt Celluloseether, Celluloseester und/oder Acrylharze verwendet werden. Besonders bevorzugt werden Ethylcellulose, Hydroxypropylmethylcellulose, Hydroxypropylcellulose, Hydroxyethylcellulose, Methylcellulose, Poly(meth)acrylsäure und/oder deren Derivate, wie deren Salze, Amide oder Ester eingesetzt.

Sofern hydrophobe Verbindungen als Retardmatrix verwendet werden, können Fettsäuren, Fettalkohole oder entsprechenden Ester oder Ether oder deren Gemische zum Einsatz kommen. Besonders bevorzugt werden als hydrophobe Verbindungen Mono- oder Diglyceride von C12-C30-Fettsäuren und/oder C12-C30-Fettalkohole und/oder Wachse oder deren Gemische eingesetzt.

Es ist auch möglich, Mischungen der vorstehend genannten hydrophilen und hydrophoben Matrix-Materialien einzusetzen.

Vorzugsweise kann die Komponente (b) als viskositätserhöhendes Mittel auch als Material für eine retardierende Matrix dienen, wenn dies der Aufbau der erfindungsgemäßen Darreichungsform zulässt.

Des weiteren kann auch die Komponente (C) und die ggf. vorhandene Komponente (D), die zur Erzielung der erfindungsgemäß notwendigen Bruchfestigkeit von mindestens 500 N, vorzugsweise mindestens 750 N, dienen, ggf. als zusätzliche retardierende Matrixmaterialien dienen.

Entsprechende retardierende Verbindungen und Verfahren zur Retardierung den erfindungsgemäßen Darreichungsformen sowie zum Aufbringen magensaftresistenter Überzüge sind dem Fachmann beispielsweise aus "Coated Pharmaceutical Dosage Forms - Fundamentals, Manufacturing Techniques, Biopharmaceutical Aspects, Test Methods and Raw Materials" von Kurt H. Bauer, K. Lehmann, Hermann P. Osterwald, Rothgang, Gerhart, 1. Auflage, 1998, Medpharm Scientific Publishers bekannt.

Die offenbarten Darreichungsformen eignen sich zur oralen, vaginalen oder rektalen, vorzugsweise zur oralen, 1 x täglichen Einnahme bei Mensch und Tier.

Die offenbarte Darreichungsform kann in multipartikulärer Form, bevorzugt in Form von Mikrotabletten, Mikropellets, Granulaten, Sphäroiden, Perlen oder Pellets, ggf. in Kapseln abgefüllt oder zu Tabletten verpresst, vorliegen. Vorzugsweise weisen die multipartikulären Formen eine Größe bzw. Größenverteilung im Bereich von 0,1 bis 3 mm, besonders bevorzugt im Bereich von 0,5 bis 2 mm auf. Je nach gewünschter Darreichungsform werden ggf. auch die üblichen Hilfsstoffe (B) zur Formulierung der Darreichungsform mitverwendet.

In einer besonders bevorzugten Ausführungsform liegt die offenbarte Darreichungsform in Form einer Tablette, einer Kapsel oder in Form eines oralen osmotischen therapeutischen Systems (OROS) vor, vorzugsweise wenn mindestens noch eine weitere missbrauchsverhindernde Komponente (a) - (f) vorhanden ist.

Die erfindungsgemäße, gegen Missbrauch gesicherte, feste Darreichungsform wird vorzugsweise hergestellt, indem die Komponenten (A), (C), ggf. (D), ggf. mindestens eine der zusätzlichen missbrauchsverhindernden Komponenten (a) - (f) und ggf. die weiteren Hilfsstoffe (B), wie vorzugsweise die retardierenden Matrix-Verbindungen gemischt werden, wobei die Komponenten (a)-(f), wenn notwendig, separat mit der Komponente (C) und ggf. (D) vermischt werden und die resultierende(n) Mischung(en) ggf. nach einer Granulierung zu der Darreichungsform unter vorangehender oder gleichzeitiger Wärmeeinwirkung durch Krafteinwirkung geformt wird (werden).

Die Granulierung kann gemäß einem Schmelzverfahren oder gemäß einer Feuchtgranulierung durchgeführt werden.

Diese Mischung(en) der Komponenten der erfindungsgemäßen Darreichungsform kann (können) in einem dem Fachmann bekannten Mischgerät erfolgen. Das Mischgerät kann beispielsweise ein Wälzmischer, Schüttelmischer, Schermischer oder Zwangsmischer sein.

Die resultierende(n) Mischung(en) wird vorzugsweise direkt durch Krafteinwirkung zu der erfindungsgemäßen Darreichungsform unter vorangehender und/oder gleichzeitiger Wärmeeinwirkung geformt. Beispielsweise kann die Mischung durch Direkttablettierung zu Tabletten geformt werden. Bei einer Direkttablettierung unter gleichzeitiger Wärmeeinwirkung wird das Tablettierwerkzeug, d. h. Unterstempel, Oberstempel und Matrize, zumindest bis zur Erweichung des Polymeren (C) kurz erhitzt und dabei verpresst. Bei einer Direkttablettierung unter vorangehender Wärmeeinwirkung wird das zu verpreßende Gut unmittelbar vor der Tablettierung mindestens bis zur Erweichungstemperatur der Komponente (C) erhitzt und anschließend gepresst.

Die resultierende(n) Mischung(en) aus den Komponenten (A), (C), ggf. (D), den ggf. vorhandenen Komponenten (a) - (f) und ggf. weiteren Hilfsstoffe (B), insbesondere den retardierenden Matrix-Verbindungen, kann (können) auch zuerst granuliert, und anschließend unter vorangehender oder gleichzeitiger Wärmeeinwirkung zu der erfindungsgemäßen Darreichungsform unter Krafteinwirkung geformt werden.

Es ist auch möglich, die resultierende Mischung enthaltend den Wirkstoff und/oder eines oder mehrere seiner pharmazeutisch akzeptablen Salzen (A) sowie ggf. physiologisch verträgliche Hilfsstoffe (B) wie die Komponenten (a) bis (f) und ggf. der retardierenden Matrix-Verbindungen und mindestens ein synthetisches oder natürliches Polymer (C) und gegebenenfalls ein Wachs (D), unter einer Krafteinwirkung zu der Darreichungsform zu formen, gegebenenfalls die Formlinge zu vereinzeln und gegebenenfalls jeweils nach Größen zu separieren und nach oder während einer Erwärmung bis wenigstens zum Erweichungspunkt der Komponente (C) solange unter einer Krafteinwirkung zu belassen, bis die Formlinge eine Bruchhärte von mindestens 500 N, vorzugsweise mindestens 750 N, aufweisen, gegebenenfalls mit einer Umhüllung ggf. mit einem retardierenden Überzug zu versehen und die Formlinge gegebenenfalls alle wieder zu vermischen. Eine solche Verfahrensweise ist auch Gegenstand der internationalen Patentanmeldung PCT/EP2004/014679. Dem Fachmann ist geläufig, dass dabei durch mit Verwendung von Antioxidantien ggf. auf die Einhaltung einer Inertgasatmosphäre während des Herstellungsverfahrens verzichtet werden kann.

Darüber hinaus kann die notwendige Erwärmung der Mischung und/oder die Formlinge vor oder während der notwendigen Krafteinwirkung zur Erzielung der erfindungsgemäßen Bruchfestigkeit bzw. Härte von mindestens 500 N, vorzugsweise 750 N, mit Hilfe von Ultraschall erreicht werden. Eine entsprechende Verfahrensweise ist in der internationalen Patentanmeldung PCT/EP2005/004225. offenbart.

Sofern die Komponenten (c) und/oder (d) und/oder (f) in der erfindungsgemäßen Darreichungsform vorhanden sind, ist darauf zu achten, dass sie so formuliert oder so gering dosiert sind, dass sie bei bestimmungsgemäßer Applikation der Darreichungsform praktisch keine den Patienten oder die Wirksamkeit der (des) Opioide(s) beeinträchtigende Wirkung entfalten können.

Sofern die erfindungsgemäße Darreichungsform die Komponente (d) und/oder (f) enthält, ist die Dosierung so zu wählen, dass bei bestimmungsgemäßer oraler Applikation keine negative Wirkung hervorgerufen wird. Wird jedoch die vorgesehene Dosierung der Darreichungsform versehentlich, insbesondere durch Kinder, oder beim Missbrauch überschritten, wird Übelkeit bzw. Brechreiz bzw. schlechter Geschmack hervorgerufen. Die jeweilige Menge der Komponente (d) und/oder (f), die vom Patienten bei bestimmungsgemäßer oraler Applikation noch toleriert wird, kann vom Fachmann durch einfache Vorversuche ermittelt werden.

Sofern aber unabhängig von der praktisch nicht möglichen Pulverisierbarkeit der erfindungsgemäßen Darreichungsform zur Sicherung der Darreichungsform enthaltend die Komponenten (c) und/oder (d) und/oder (f) vorgesehen ist, sollten diese Komponenten bevorzugt in einer so hohen Dosierung zum Einsatz kommen, dass sie bei einer mißbräuchlichen Applikation der Darreichungsform eine intensive negative Wirkung beim Missbraucher hervorrufen. Dies gelingt vorzugsweise durch eine räumliche Trennung zumindest der (des) Opioide(s) von den Komponenten (c) und/oder (d) und/oder (f), wobei bevorzugt das (die) Opioid(e) in wenigstens einer Untereinheit (X) und die Komponenten (c) und/oder (d) und/oder (f) in wenigstens einer Untereinheit (Y) vorliegen, und wobei die Komponenten (c), (d) und (f) bei bestimmungsgemäßer Applikation der Darreichungsform bei Einnahme und/oder im Körper nicht ihre Wirkung entfalten und die übrigen Formulierungskomponenten insbesondere die Komponente (C) und ggf. (D)identisch sind.

Sofern die erfindungsgemäße Darreichungsform wenigstens 2 der Komponenten (c) und (d) bzw. (f) aufweist, können diese jeweils in derselben oder in verschiedenen Untereinheiten (Y) vorliegen. Vorzugsweise liegen, sofern vorhanden, alle Komponenten (c) und (d) und (f) in ein- und derselben Untereinheit (Y) vor.

Bei einer räumlichen Trennung in Untereinheit(en) (X) und Untereinheit(en) (Y) und unabhängig von der Anordnung dieser Untereinheiten in der Darreichungsform, enthält eine Untereinheit (X) den Wirkstoff in retardierter Form, so dass dieser in einer kontrollierten Freisetzung eine einmal tägliche Verabreichung gewährleistet. Untereinheiten im Sinne der vorliegenden Erfindung sind feste Formulierungen, die jeweils neben üblichen, dem Fachmann bekannten Hilfsstoffen das (die) Opioid(e), mindestens ein Polymer (C) und gegebenenfalls wenigstens eine der gegebenenfalls vorhandenen Komponenten (a) und/oder (b) und/oder (e) bzw. jeweils wenigstens ein Polymer (C) und den (die) Antagonist(en) und/oder das Emetikum (die Emetika) und/oder die Komponente (e) und/oder die Komponente (f) und gegebenenfalls wenigstens eine der gegebenenfalls vorhandenen Komponenten (a) und/oder (b) sowie ggf. die retardierenden Matrix-Verbindungen enthalten. Dabei ist darauf zu achten, dass jede der Untereinheiten nach dem vorstehend angegebenen Verfahren formuliert werden.

Ein wesentlicher Vorteil der getrennten Formulierung der (des) Opioide(s) von den Komponenten (c) bzw. (d) bzw. (f) in Untereinheiten (X) und (Y) der erfindungsgemäßen Darreichungsform besteht darin, dass bei ihrer bestimmungsgemäßen Applikation die Komponenten (c) und/oder (d) und/oder (f) bei Einnahme und/oder im Körper praktisch nicht freigesetzt werden oder nur in so geringen Mengen freigesetzt werden, dass sie keine den Patienten oder den Therapieerfolg beeinträchtigende Wirkung entfalten oder bei der Passage durch den Körper des Patienten nur an solchen Freisetzungsorten abgegeben werden, an denen eine für ihre Wirksamkeit ausreichende Resorption nicht gegeben ist. Vorzugsweise werden die Komponenten (c) und/oder (d) und/oder (f) bei bestimmungsgemäßer Applikation der Darreichungsform im Körper des Patienten praktisch nicht freigesetzt oder vom Patienten nicht wahrgenommen.

Der Fachmann versteht, dass diese vorstehend genannten Bedingungen in Abhängigkeit von den jeweils eingesetzten Komponenten (c), (d) und/oder (f) sowie der Formulierung der Untereinheiten bzw. der Darreichungsform variieren können. Die für die jeweilige Darreichungsform optimale Formulierung kann durch einfache Vorversuche ermittelt werden. Entscheidend ist, dass die jeweiligen Untereinheiten das Polymer (C) enthalten und in der angegebenen Weise formuliert wurden.

Sollte es den Missbrauchern wider Erwarten gelingen, eine solche erfindungsgemäße Darreichungsform, welche die Komponenten (c) und/oder (e) und/oder (d) und/oder (f) in Untereinheiten (Y) aufweist, zum Zwecke der mißbräuchlichen Einnahme der (des) Opioide(s) zu zerkleinern und ein Pulver zu erhalten, das mit einem geeigneten Extraktionsmittel extrahiert werden soll, wird neben dem (den) Opioid(en) auch die jeweilige Komponente (c) und/oder (e) und/oder (f) und/oder (d) in einer Form erhalten, in der sie von dem (den) Opioid(en) nicht auf einfache Weise zu separieren ist, so dass sie bei der Applikation der manipulierten Darreichungsform, insbesondere bei oraler und/oder parenteraler Verabreichung, ihre Wirkung schon bei Einnahme und/oder im Körper entfaltet und zusätzlich eine der Komponente (c) und/oder (d) und/oder (f) entsprechende negative Wirkung beim Missbraucher hervorruft oder ein Versuch, das (die) Opioid(e) zu extrahieren durch die Farbgebung abschreckt und so den Missbrauch der Darreichungsform verhindert.

Die Formulierung einer erfindungsgemäßen Darreichungsform, in der eine räumliche Trennung der (des) Opioide(s) von den Komponenten (c), (d) und/oder (e), vorzugsweise durch Formulierung in verschiedenen Untereinheiten erfolgt ist, kann in vielfältiger Art und Weise erfolgen, wobei die entsprechenden Untereinheiten in der erfindungsgemäßen Darreichungsform jeweils in beliebiger räumlicher Anordnung zueinander vorliegen können, sofern die vorstehend genannten Bedingungen für die Freisetzung der Komponenten (c) und/oder (d) einerseits und für die Freisetzung des Opioids, nämlich eine kontrollierte Freisetzung für eine einmal tägliche Verabreichung, andererseits, erfüllt sind.

Der Fachmann versteht, dass die ggf. auch vorliegenden Komponente(n) (a) und/oder (b) bevorzugt sowohl in den jeweiligen Untereinheiten (X) und (Y) als auch in Form von eigenständigen, den Untereinheiten (X) und (Y) entsprechenden Untereinheiten (Y') in der erfindungsgemäßen Darreichungsform formuliert werden können, so lange die Sicherung der Darreichungsform gegen den Missbrauch wie auch die Opioid-Freisetzung über 24 Stunden bei bestimmungsgemäßer Applikation durch die Art der Formulierung nicht beeinträchtig werden und das Polymer (C) mit formuliert und die Formulierung gemäß den vorstehend angegebenen Verfahren durchgeführt wird.

In einer bevorzugten Ausführungsform der erfindungsgemäßen Darreichungsform liegen die Untereinheiten (X) und (Y) in multipartikulärer Form vor, wobei Mikrotabletten, Mikrokapseln, Mikropellets, Granulaten, Sphäroiden, Perlen oder Pellets bevorzugt sind und sowohl für die Untereinheit (X) als auch (Y) dieselbe Form, d.h. Gestaltung gewählt wird, damit keine Separierung der Untereinheiten (X) von (Y) durch mechanische Auslese möglich ist. Die multipartikulären Formen weisen bevorzugt eine Größe im Bereich von 0,1 bis 3 mm, vorzugsweise 0,5 bis 2 mm auf.

Die Untereinheiten (X) und (Y) in multipartikulärer Form können auch bevorzugt in eine Kapsel abgefüllt oder zu einer Tablette gemäß Anspruch 1 des Patents verpresst werden, wobei die jeweiligen Endformulierungen dergestalt erfolgen, dass die Untereinheiten (X) und (Y) auch in der resultierenden Darreichungsform erhalten bleiben.

Die jeweiligen multipartikulären Untereinheiten (X) bzw. (Y) mit identischer Formgebung sollten auch nicht visuell voneinander unterscheidbar sein, damit sie vom Missbraucher nicht durch einfaches Sortieren voneinander separiert werden können. Dies kann beispielsweise durch das Aufbringen identischer Überzüge gewährleistet werden, die neben dieser Egalisierungsfunktion auch weitere Funktionen übernehmen können, wie z. B. die Retardierung eines oder mehrerer Opioide(s) oder eine magensaftresistente Ausrüstung und/oder Geschmackskaschierung der jeweiligen Untereinheiten.

In einer weiteren bevorzugten Ausführungsform der vorliegenden Erfindung sind die Untereinheiten (X) und (Y) jeweils schichtförmig zueinander angeordnet.

Bevorzugt sind hierfür die schichtförmigen Untereinheiten (X) und (Y) in der erfindungsgemäßen Darreichungsform vertikal oder horizontal zueinander angeordnet, wobei jeweils auch eine oder mehrere schichtförmige Untereinheiten (X) und eine oder mehrere schichtförmige Untereinheiten (Y) in der Darreichungsform vorliegen können, so dass neben den bevorzugten Schichtenfolgen (X)-(Y) bzw. (X)-(Y)-(X) beliebige andere Schichtenfolgen in Betracht kommen, ggf. in Kombination mit Schichten enthaltend die Komponenten (a) und/oder (b).

Ebenfalls bevorzugt ist eine erfindungsgemäße Darreichungsform, in der die Untereinheit (Y) einen Kern bildet, der von der retardierten Untereinheit (X) vollständig umhüllt wird, wobei zwischen diesen Schichten eine Trennschicht (Z) vorhanden sein kann. Ein entsprechender Aufbau eignet sich bevorzugt auch für die vorstehend genannten multipartikulären Formen, wobei dann beide Untereinheiten (X) und (Y) sowie eine ggf. vorhandene Trennschicht (Z), die der erfindungsgemäßen Härteanforderung genügen muss, in ein- und derselben multipartikulären Form formuliert sind.

In einer weiteren bevorzugten Ausführungsform der erfindungsgemäßen Darreichungsform bildet die Untereinheit (X) einen Kern, der von der Untereinheit (Y) umhüllt wird, wobei letztere wenigstens einen Kanal aufweist, der von dem Kern an die Oberfläche der Darreichungsform führt.

Zwischen einer Schicht der Untereinheit (X) und einer Schicht der Untereinheit (Y) kann die erfindungsgemäße Darreichungsform jeweils eine oder mehrere, vorzugsweise eine, ggf. quellbare Trennschicht (Z) zur räumlichen Trennung der Untereinheit (X) von (Y) aufweisen.

Sofern die offenbarte Darreichungsform die schichtförmigen Untereinheiten (X) und (Y) sowie eine ggf. vorhandene Trennschicht (Z) in einer zumindest teilweise vertikalen oder horizontalen Anordnung aufweist, liegt sie bevorzugt in Form einer Tablette, eines Coextrudats oder Laminats vor.

Hierbei kann in einer besonders bevorzugten Ausführungsform die freie Oberfläche der Untereinheit (Y) vollständig und ggf. zumindest ein Teil der freien Oberfläche der Untereinheit(en) (X) und ggf. zumindest ein Teil der freien Oberfläche der ggf. vorhandenen Trennschicht(en) (Z) mit wenigstens einer die Freisetzung der Komponente (c) und/oder (e) und/oder (d) und/oder (f) verhindernden Barriereschicht (Z') überzogen sein. Auch die Barriereschicht (Z') muss die erfindungsgemäßen Härtevoraussetzungen erfüllen.

Ebenfalls besonders bevorzugt ist eine Ausführungsform der erfindungsgemäßen Darreichungsform, die eine vertikale oder horizontale Anordnung der Schichten der Untereinheiten (X) und (Y) und wenigstens eine dazwischen angeordnete Push-Schicht (p) sowie ggf. eine Trennschicht (Z) aufweist, in der sämtliche freie Oberflächen des aus den Untereinheiten (X) und (Y), der Push-Schicht und der ggf. vorhandenen Trennschicht (Z) bestehenden Schichtaufbaus mit einem semipermeablen Überzug (E) ausgerüstet sind, der für ein Freisetzungsmedium, d.h. üblicherweise eine physiologische Flüssigkeit, durchlässig, für das (die) Opioid(e) und für die Komponente (c) und/oder (d) und/oder (f) im wesentlichen undurchlässig ist, und wobei dieser Überzug (E) im Bereich der Untereinheit (X) wenigstens eine Öffnung zur Freisetzung der (des) Opioide(s) aufweist.

Eine entsprechende Darreichungsform ist dem Fachmann beispielsweise unter der Bezeichnung orales osmotisches therapeutisches System (OROS), ebenso wie geeignete Materialien und Verfahren zu dessen Herstellung, u.a. aus US 4,612,008, US 4,765,989 und US 4,783,337 bekannt.

Aus dem Stand der Technik ist dem Fachmann ebenfalls eine osmotische Darreichungsform enthaltend ein analgetisches Opioid und einen Farbstoff als aversives Mittel bekannt (WO 03/015531). Vorzugsweise besteht der Tablettenkem aus zwei Schichten, eine opioidhaltigen Schicht und eine Push-Schicht, wobei die Push-Schicht den Farbstoff als aversives Mittel enthält.

In einer weiteren bevorzugten Ausführungsform der beanspruchten Erfindung hat die Untereinheit (X) der erfindungsgemäßen Darreichungsform die Form einer Tablette, deren Steg und ggf. eine der beiden Grundflächen mit einer die Komponente (c) und/oder (d) und/oder (f) enthaltenden Barriereschicht (Z') bedeckt ist.

Der Fachmann versteht, dass die bei der Formulierung der erfindungsgemäßen Darreichungsform jeweils zum Einsatz kommenden Hilfsstoffe der Untereinheit(en) (X) bzw. (Y) sowie ggf. der vorhandenen Trennschicht(en) (Z) und/oder der Barriereschicht(en) (Z') in Abhängigkeit von deren Anordnung in der erfindungsgemäßen Darreichungsform, der Applikationsart sowie in Abhängigkeit von dem jeweiligen Opioid, der ggf. vorhandenen Komponenten (a) und/oder (b) und/oder (e) und der Komponente (c) und/oder (d) und/oder (f) unter Einhaltung der Freisetzung des Wirkstoffes über 24 Stunden variieren. Die Materialien, die über die jeweils erforderlichen Eigenschaften verfügen sind, dem Fachmann an sich bekannt.

Sofern die Freisetzung der Komponente (c) und/oder (d) und/oder (f) aus der Untereinheit (Y) der erfindungsgemäßen Darreichungsform mit Hilfe einer Umhüllung, vorzugsweise einer Barriereschicht, verhindert wird, kann die Untereinheit aus üblichen, dem Fachmann bekannten Materialien bestehen, sofern sie wenigstens ein Polymer( C) zur Erfüllung der Härtebedingung der erfindungsgemäßen Darreichungsform enthält.

Ist eine entsprechende Barriereschicht (Z') zur Verhinderung der Freisetzung der Komponente (c) und/oder (d) und/oder (f) nicht vorgesehen, sind die Materialien der Untereinheiten so zu wählen, dass eine Freisetzung der jeweiligen Komponente (c) und/oder (d) aus der Untereinheit (Y) praktisch ausgeschlossen ist.

Bevorzugt können hierzu die nachstehend aufgeführten Materialien zum Einsatz kommen, die auch für den Aufbau der Barriereschicht geeignet sind. Bevorzugte Materialien sind solche, die ausgewählt sind aus der Gruppe umfassend Alkylcellulosen, Hydroxyalkylcellulosen, Glucane, Skleroglucane, Mannane, Xanthane, Copolymere aus Poly[bis(p-carboxyphenoxy)propan und Sebacinsäure, vorzugsweise in einem Molverhältnis von 20:80 (unter der Bezeichnung Polifeprosan 20^{®} am Markt geführt), Carboxymethylcellulosen, Celluloseether, Celluloseester, Nitrocellulose, Polymere auf Basis von (Meth)acrylsäure sowie deren Estern, Polyamide, Polycarbonate, Polyalkylene, Polyalkylenglykole, Polyalkylenoxide, Polyalkylenterephtalate, Polyvinylalkohole, Polyvinylether, Polyvinylester, halogenierte Polyvinyle, Polyglykolide, Polysiloxane sowie Polyurethane und deren Copolymeren oder Mischungen.

Besonders geeignete Materialien können ausgewählt werden aus der Gruppe umfassend Methylcellulose, Ethylcellulose, Hydroxypropylcellulose, Hydroxypropylmethylcellulose, Hydroxybutylmethylcellulose, Celluloseacetat, Cellulosepropionat (von niederem, mittlerem oder erhöhtem Molekulargewicht), Celluloseacetatpropionat, Celluloseacetatbutyrat, Celluloseacetatphtalat, Carboxymethylcellulose, Cellulosetriacetat, Natrium-Cellulosesulfat, Polymethylmethacrylat, Polyethylmethacrylat, Polybutylmethacrylat, Polyisobutylmethacrylat, Polyhexylmethacrylat, Polyisodecylmethacrylat, Polylaurylmethacrylat, Polyphenylmethacrylat, Polymethylacrylat, Polyisopropylacrylat, Polyisobutylacrylat, Polyoctatdecylacrylat, Polyethylen, Polyethylen niederer Dichte, Polyethylen hoher Dichte, Polypropylen, Polyethylenglykol, Polyethylenoxid, Polyethylenterephtalat, Polyvinylalkohol, Polyvinylisobutylether, Polyvinylacetat und Polyvinylchlorid.

Besonders geeignete Copolymere können ausgewählt werden aus der Gruppe umfassend Copolymere aus Butylmethacrylat und Isobutylmethacrylat, Copolymere aus Methylvinylether und Maleinsäure mit erhöhtem Molekulargewicht, Copolymere aus Methylvinylether und Maleinsäuremonoethylester, Copolymere aus Methylvinylether und Maleinsäureanhydrid sowie Copolymere aus Vinylalkohol und Vinylacetat.

Weitere zur Formulierung der Barriereschicht geeignete Materialien sind Stärke gefülltes Polycaprolacton (WO98/20073) aliphatische Polyesteramide (DE 19 753 534 A1, DE 19 800 698 A1, EP 0 820 698 A1), aliphatische und aromatische Polyesterurethane (DE 19822979), Polyhydroxyalkanoate, insbesondere Polyhydroxybutyrate, Polyhydroxyvaleriate), Casein (DE 4 309 528), Polylactide und Copolylactide (EP 0 980 894 A1).

Ggf. können die vorstehend genannten Materialien mit weiteren üblichen, dem Fachmann bekannten Hilfsstoffen, vorzugsweise ausgewählt aus der Gruppe bestehend aus Glycerylmonostearat, halbsynthetischen Triglyceridderivaten, halbsynthetischen Glyceriden, hydriertem Rizinusöl, Glycerylpalmitostearat, Glycerylbehenat, Polyvinylpyrrolidon, Gelatine, Magnesiumstearat, Stearinsäure, Natriumstearat, Talkum, Natriumbenzoat, Borsäure und kolloidalem Silica, Fettsäuren, substituierten Triglyceriden, Glyceriden, Polyoxyalkylenglykolen und deren Derivate abgemischt werden.

Sofern die erfindungsgemäße Darreichungsform eine Trennschicht (Z') aufweist, kann diese, ebenso wie die nicht umhüllte Untereinheit (Y) vorzugsweise aus den vorstehend, für die Barriereschicht beschriebenen Materialien bestehen. Der Fachmann versteht, dass auch über die Dicke der Trennschicht die Freisetzung der (des) Opioide(s) und/oder Opiate(s) bzw. der Komponente (c) und/oder (d) aus der jeweiligen Untereinheit mit gesteuert werden kann.

Die erfindungsgemäße Darreichungsform weist eine kontrollierte Freisetzung des Wirkstoffes über wenigstens 24 Stunden auf und eignet sich so zur 1 x täglichen Verabreichung.

### Methode zur Bestimmung der Bruchfestigkeit

Zur Überprüfung, ob ein Material als Komponente (C) oder (D) eingesetzt werden kann, wird das Material zu einer Tablette mit einem Durchmesser von 10 mm und einer Höhe von 5mm mit einer Kraft von 150 N, bei einer Temperatur entsprechend mindestens dem Erweichungspunkt des Polymeren und bestimmt mit Hilfe eines DSC-Diagramms des Materials verpresst. Mit so hergestellten Tabletten wird gemäß der Methode zur Bestimmung der Bruchfestigkeit von Tabletten, veröffentlicht im Europäischen Arzneibuch 1997, Seite 143, 44, Methode Nr. 2.9.8. unter Einsatz der nachstehend aufgeführten Apparatur die Bruchfestigkeit bestimmt. Als Apparatur für die Messung wird eine Zwick Materialprüfmaschine "Zwick Z 2.5", Materialprüfmaschine Fmax 2.5 kN mit einem Traversenweg von max. 1150 mm, der durch einen Aufbau mit Hilfe einer Säule und einer Spindel einzustellen ist, einen freien Arbeitsraum nach hinten von 100 mm und einer zwischen 0,1 bis 800 mm /min. einstellbaren Prüfgeschwindigkeit und einer Software: testControl eingesetzt. Es wird ein Druckstempel mit schraubbaren Einsätzen und einem Zylinder (Durchmesser 10 mm), ein Kraftaufnehmer, Fmax. 1 kN, Durchmesser 8 mm, Klasse 0.5 ab 10 N, Klasse 1 ab 2 N nach ISO 7500-1, mit Hersteller-Prüfzertifikat M nach DIN 55350-18 (Zwick-Bruttokraft Fmax 1,45 kN) zur Messung eingesetzt (alles Apparaturen der Firma Zwick GmbH & Co. KG, Ulm, Deutschland) mit der Bestell-Nr. BTC-FR 2.5 TH. D09 für die Prüfmaschine, der Bestell-Nr. BTC-LC 0050N. P01 für den Kraftaufnehmer, der Bestell-Nr. BO 70000 S06 für die Zentriervorrichtung.

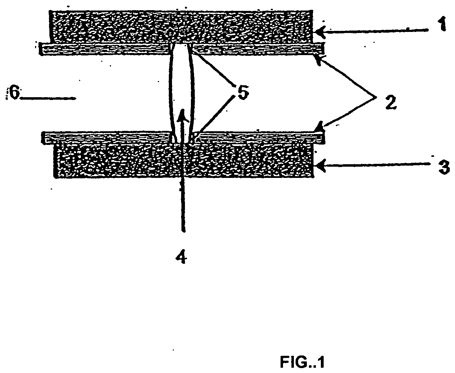

**Figur 1** zeigt die Messung der Bruchfestigkeit einer Tablette, insbesondere die dafür eingesetzte Justierungsvorrichtung (6) der Tablette (4) vor und während der Messung. Dazu wird die Tablette (4) zwischen der oberen Druckplatte (1) und der unteren Druckplatte (3) der nicht dargestellten Vorrichtung zur Kraftaufbringung mit Hilfe von zwei 2-teiligen Einspannvorrichtungen, die jeweils mit der oberen bzw. unteren Druckplatte nach Einstellung des zur Aufnahme und zur Zentrierung der zu messenden Tablette notwendigen Abstands (5) fest verbunden (nicht dargestellt) werden. Zur Einstellung des Abstands (5) können die 2-teiligen Einspannvorrichtungen jeweils auf der Druckplatte, auf der sie gelagert sind, horizontal nach außen oder innen bewegt werden.

Als bruchfest bei einer bestimmten Krafteinwirkung werden auch die Tabletten eingestuft, bei denen kein Bruch feststellbar, aber ggf. eine plastische Verformung der Tablette durch die Krafteinwirkung erfolgt ist.

Bei den erfindungsgemäß erhaltenen Darreichungsformen wird die Bruchfestigkeit nach der aufgeführten Meßmethode bestimmt, wobei von Tabletten abweichenden Darreichungsformen ebenso geprüft werden.

Im Folgenden wird die Erfindung anhand von Beispielen erläutert. Diese Erläuterungen sind lediglich beispielhaft und schränken den allgemeinen Erfindungsgedanken nicht ein.

### Beispiel 1

### a) Herstellung einer gegen Missbrauch gesicherten Oxycodon-haltigen Tablette

Die in der Tabelle 1 aufgeführten Mengen an Oxycodon Hydrochlorid, Polyethylenoxidpulver und Hydroxypropylmethylcellulose (Metholose 90 SH 100 000) als Retardmatrixmaterial wurden in einem Freifallmischer gemischt. Das Tablettierwerkzeug, das aus Matrize, Ober- und Unterstempel mit einem Durchmesser von 10 mm besteht, wurde in einem Heizschrank auf 90°C erhitzt. Mittels des erhitzten Werkzeug wurden jeweils 600 mg der Pulvermischung verpresst, wobei der Pressdruck für mindestens 15 Sekunden aufrechterhalten wurde.

**Tabelle 1**

| Komponenten | Pro Tablette | Gesamtansatz |
|---|---|---|
| Oxycodon HCl | 80,0 mg | 40,0 g |
| Polyethylenoxid, NF, MG 7 000 000 (Polyox WSR 303, Dow Chemicals) | 470,0 mg | 235,0 g |
| Hydroxypropylmethylcellulose 100 000 mPos (Metholose 90 SH 100 000) | 50,0 mg | 25,0 g |
| Gesamtgewicht | 600,0 mg | 300,0 g |

Die Bruchfestigkeit der Tabletten wird nach der vorstehend beschriebenen Methode bestimmt. Bei der Krafteinwirkung von 500 N trat kein Bruch auf. Die Tabletten konnten weder mit einem Hammer noch mit Hilfe von Mörser oder Pistill zerkleinert werden.

### In-Vitro Freisetzung aus den nach a) hergestellten Tabletten

Die In-Vitro Freisetzung von Oxycodon Hydrochlorid aus den nach a) hergestellten Tabletten wurde in einer Blattrührerapparatur mit Sinker gemäß der in dem Europäischen Arzneibuch (European Pharmacopeia) beschriebenen Methode, bestimmt. Die Temperatur des Freisetzungsmediums betrug 37°C und die Umdrehungsgeschwindigkeit des Rührers 75 min⁻¹. Als Freisetzungsmedium wurden 600 ml Darmsaft pH 6,8 verwendet. Die jeweils zu einem Zeitpunkt im Lösungsmedium freigesetzte Menge an Oxycodon wurde spektralphotometrisch bestimmt. Die prozentuale freigesetzte Menge, bezogen auf die Gesamtmenge Oxycodon Hydrochlorid, zu jedem Zeitpunkt ist in Tabelle 2 dargestellt.

**Tabelle 2**

| Zeit, min | Freigesetzte Menge, Gew.-% |
|---|---|
| 30 | 11 |
| 240 | 40 |
| 480 | 61 |
| 720 | 76 |
| 1080 | 92 |
| 1440 | 97 |

## Patentansprüche

1. Gegen Missbrauch gesicherte, orale Darreichungsform mit kontrollierter Opioid Freisetzung zur Anwendung bei der Schmerzbekämpfung über eine Dauer von mindestens 24 Stunden, **dadurch gekennzeichnet, dass** sie **in Form einer Tablette vorliegt,** wenigstens ein Opioid mit Missbrauchspotential (A) und/oder einer seiner physiologisch verträglichen Verbindungen, mindestens ein synthetisches oder natürliches Polymer (C), ggf. wenigstens ein retardierendes Matrix-Material, ggf. wenigstens einen retardierenden Überzug, ggf. wenigstens einen physiologisch verträglichen Hilfsstoff (B) und ggf. wenigstens ein Wachs (D) umfasst, wobei die Komponente (C) bzw. (D) jeweils eine Bruchfestigkeit von mindestens 500 N aufweist, wobei die Komponente(n) (C) und gegebenenfalls (D) in solchen Mengen vorliegt, dass die Darreichungsform eine Bruchfestigkeit von mindestens 500 N aufweist, und wobei die Verabreichung einmal täglich erfolgt;
und wobei die Bruchfestigkeit der Komponente (C) bzw. (D) wie folgt bestimmt wird: das Material wird zu einer Tablette mit einem Durchmesser von 10 mm und einer Höhe von 5 mm mit einer Kraft von 150 N, bei einer Temperatur entsprechend mindestens dem Erweichungspunkt, bestimmt mit Hilfe eines DSC-Diagramms des Materials, verpresst; mit so hergestellten Tabletten wird gemäß der Methode zur Bestimmung der Bruchfestigkeit von Tabletten, veröffentlicht im Europäischen Arzneibuch 1997, Seite 143, 44, Methode Nr. 2.9.8. unter Einsatz der nachstehend aufgeführten Apparatur die Bruchfestigkeit bestimmt; als Apparatur für die Messung wird eine Zwick Materialprüfmaschine "Zwick Z 2.5", Materialprüfmaschine Fmax 2.5 kN mit einem Traversenweg von max. 1150 mm, der durch einen Aufbau mit Hilfe einer Säule und einer Spindel einzustellen ist, einen freien Arbeitsraum nach hinten von 100 mm und einer zwischen 0,1 bis 800 mm /min. einstellbaren Prüfgeschwindigkeit und einer Software: testControl eingesetzt; es wird ein Druckstempel mit schraubbaren Einsätzen und einem Zylinder, Durchmesser 10 mm, ein Kraftaufnehmer, Fmax. 1 kN, Durchmesser 8 mm, Klasse 0.5 ab 10 N, Klasse 1 ab 2 N nach ISO 7500-1, mit Hersteller-Prüfzertifikat M nach DIN 55350-18, Zwick-Bruttokraft Fmax 1,45 kN, zur Messung eingesetzt.

2. Darreichungsform zur Anwendung gemäß Anspruch 1, **dadurch gekennzeichnet, dass** das Opioid wenigstens ein Opioid ausgewählt aus der Gruppe umfassend Oxycodon, Hydromorphon, Morphin, Oxymorphon, Tramadol, deren Stereoisomeren, deren Racemate, deren Enantiomeren, deren Diastereomeren in beliebigen Mischungen, deren physiologisch verträgliche Verbindungen, vorzugsweise physiologisch verträgliche Salze, ganz besonders bevorzugt Hydrochloride und Solvate, und deren Derivate, vorzugsweise Ester, Ether oder Amide ist.

3. Darreichungsform zur Anwendung gemäß Anspruch 1, **dadurch gekennzeichnet, dass** als Opioid wenigstens ein Opioid ausgewählt aus der Gruppe umfassend (2R, 3R)-1-Dimethylamino-3-(3-methoxy-phenyl)-2-methyl-pentan-3-ol, (1RS,3RS,6RS)-6-Dimethylaminomethyl-1-(3-methoxy-phenyl)-cyclohexan-1,3-diol, (1R,2R)-3-(2-Dimethylaminonethyl-cyclohexyl)-phenol, deren physiologisch verträglichen Salze, vorzugsweise Hydrochloride, physiologisch verträgliche Enantiomeren, Stereoisomeren, Diastereomeren und Racemate und deren physiologisch verträglichen Derivate, vorzugsweise Ether, Ester oder Amide vorliegt.

4. Darreichungsform zur Anwendung gemäß einem der Ansprüche 1 bis 3, **dadurch gekennzeichnet, dass** das Polymer (C) wenigstens ein Polymer ausgewählt aus der Gruppe umfassend Polyalkylenoxide, Polyethylene, Polypropylene, Polyvinylchloride, Polycarbonate, Polystyrole, Poly(meth)acrylate, deren Copolymerisate und Mischungen aus wenigstens zwei Vertretern der genannten Polymerklassen oder Polymeren ist.

5. Darreichungsform zur Anwendung gemäß Anspruch 4, **dadurch gekennzeichnet, dass** das Polyalkylenoxid ein Polymethylenoxid, Polyethylenoxid oder Polypropylenoxid ist.

6. Darreichungsform zur Anwendung nach einem der Ansprüche 1 bis 5, **dadurch gekennzeichnet, dass** als Polymer (C) ein hochmolekulares Polyethylenoxid vorliegt.

7. Darreichungsform zur Anwendung gemäß einem der Ansprüche 1 bis 6, **dadurch gekennzeichnet, dass** das Polymer (C) ein wasserlösliches oder wasserquellbares Polymer ist.

8. Darreichungsform zur Anwendung gemäß einem der Ansprüche 1 bis 7, **dadurch gekennzeichnet, dass** das Polyethylenoxid (C) ein Molekulargewicht von mindestens 0,5 Mio. aufweist.

9. Darreichungsform zur Anwendung gemäß Anspruch 8, **dadurch gekennzeichnet, dass** das Molekulargewicht des Polyethylenoxids (C) 1-15 Mio. beträgt.

10. Darreichungsform zur Anwendung gemäß einem der Ansprüche 1 bis 9, **dadurch gekennzeichnet, dass** als Wachs (D) wenigstens ein natürliches, halbsynthetisches oder synthetisches Wachs mit einem Erweichungspunkt von wenigstens 60°C vorliegt.

11. Darreichungsform zur Anwendung gemäß Anspruch 10, **dadurch gekennzeichnet, dass** das Wachs (D) Carnaubawachs oder Bienenwachs ist.

12. Darreichungsform zur Anwendung gemäß einem der Ansprüche 1 bis 11, **dadurch gekennzeichnet, dass** das Opioid in einer retardierenden Matrix vorliegt.

13. Darreichungsform zur Anwendung gemäß Anspruch 12, **dadurch gekennzeichnet, dass** die Komponente (C) und/oder die Komponente (D) auch als retardierende Matrix-Komponente dient.

14. Darreichungsform zur Anwendung gemäß einem der Ansprüche 1 bis 13, **dadurch gekennzeichnet, dass** wenigstens ein Hilfsstoff (B) als Material für die retardierende Matrix dient.

15. Darreichungsform zur Anwendung gemäß einem der Ansprüche 1 bis 14, **dadurch gekennzeichnet, dass** sie einen Überzug, vorzugsweise einen retardierenden Überzug, aufweist.

16. Darreichungsform zur Anwendung gemäß einem der Ansprüche 1 bis 15, **dadurch gekennzeichnet, dass** sie als Hilfsstoff (B) wenigstens eine der nachfolgenden weiteren missbrauchsverhindernden Komponenten (a)-(f) aufweist:
(a) wenigstens einen den Nasen- und/oder Rachenraum reizenden Stoff,
(b) wenigstens ein viskositätserhöhendes Mittel, das in einem mit Hilfe einer notwendigen Mindestmenge an einer wässrigen Flüssigkeit, vorzugsweise als ein aus der Darreichungsform gewonnenen Extrakt, ein Gel bildet, welches vorzugsweise beim Einbringen in eine weitere Menge einer wässrigen Flüssigkeit visuell unterscheidbar bleibt,
(c) wenigstens einen Antagonisten für den Wirkstoff mit Missbrauchspotential,
(d) wenigstens ein Emetikum,
(e) wenigstens einen Farbstoff als aversives Mittel,
(f) wenigstens einen Bitterstoff.

17. Darreichungsform zur Anwendung gemäß Anspruch 16, **dadurch gekennzeichnet, dass** der Reizstoff gemäß Komponente (a) ein Brennen, einen Juckreiz, einen Niesreiz, eine vermehrte Sekretbildung oder eine Kombination mindestens zweier dieser Reize verursacht.

18. Darreichungsform zur Anwendung gemäß Anspruch 16 oder 17, **dadurch gekennzeichnet, dass** der Reizstoff gemäß Komponente (a) auf einem oder mehreren Inhaltsstoffen wenigstens einer Scharfstoffdroge basiert.

19. Darreichungsform zur Anwendung gemäß Anspruch 18, **dadurch gekennzeichnet, dass** die Scharfstoffdroge wenigstens eine Droge ausgewählt aus der Gruppe umfassend Allii sativi Bulbus, Asari Rhizoma c. Herba, Calami Rhizoma, Capsici Fructus (Paprika), Capsici Fructus acer (Cayennepfeffer), Curcumae longae Rhizoma, Curcumae xanthorrhizae Rhizoma, Galangae Rhizoma, Myristicae Semen, Piperis nigri Fructus (Pfeffer), Sinapis albae (Erucae) Semen, Sinapis nigri Semen, Zedoariae Rhizoma und Zingiberis Rhizoma, besonders bevorzugt wenigstens eine Droge ausgewählt aus der Gruppe umfassend Capsici Fructus (Paprika), Capsici Fructus acer (Cayennepfeffer) und Piperis nigri Fructus (Pfeffer) ist.

20. Darreichungsform zur Anwendung gemäß Anspruch 18 oder 19, **dadurch gekennzeichnet, dass** der Inhaltsstoff der Scharfstoffdroge als eine o-Methoxy(Methyl)-phenol-Verbindung, eine Säureamid-Verbindung vorliegt, ein Senföl oder eine Sulfidverbindung ist oder sich von einer solchen Verbindung ableitet.

21. Darreichungsform zur Anwendung gemäß einem der Ansprüche 18 bis 20, **dadurch gekennzeichnet, dass** der Inhaltsstoff der Scharfstoffdroge wenigstens ein Inhaltsstoff ausgewählt aus der Gruppe umfassend Myristicin, Elemicin, Isoeugenol, β-Asaron, Safrol, Gingerolen, Xanthorrhizol, Capsaicinoiden, vorzugsweise Capsaicin, Piperin, vorzugsweise trans-Piperin, Glucosinolaten, vorzugsweise auf Basis von nichtflüchtigen Senfölen, besonders bevorzugt auf Basis von p-Hydroxybenzylsenföl, Methylmercaptosenföl oder Methylsulfonylsenföl, und eine von diesen Inhaltsstoffen abgeleiteten Verbindung ist.

22. Darreichungsform zur Anwendung gemäß einem der Ansprüche 16 bis 21, **dadurch gekennzeichnet, dass** die Komponente (b) wenigstens ein viskositätserhöhendes Mittel ausgewählt aus der Gruppe umfassend mikrokristalline Cellulose mit 11 Gew.% Carboxymethylcellulose-Natrium (Avicel^{®} RC 591), Carboxymethylcellulose-Natrium (Blanose^{®}, CMC-Na C300P^{®},Frimulsion BLC-5^{®}, Tylose C300 P^{®}), Polyacrylsäure (Carbopol^{®} 980 NF, Carbopol^{®} 981), Johannisbrotkernmehl (Cesagum^{®} LA-200, Cesagum^{®} LID/150, Cesagum^{®} LN-1), Pektine aus Citrusfrüchten oder Äpfeln (Cesapectin^{®} HM Medium Rapid Set), Wachsmaisstärke (C*Gel 04201^{®}), Natriumalginat (Frimulsion ALG (E401)^{®}), Guarkernmehl (Frimulsion BM^{®}, Polygum 26/1-75^{®}), lota-Carrageen (Frimulsion D021^{®}), Karaya Gummi, Gellangummi (Kelcogel F^{®}, Kelcogel LT100^{®}), Galaktomannan (Meyprogat 150 ^{®}), Tarakernmehl (Polygum 43/1^{®}), Propylenglykoalginat (Protanal-Ester SD-LB^{®}), Apfelpektin, Natrium-Hyaluronat, Tragant, Taragummi (Vidogum SP 200^{®}), fermentiertes Polysaccharid- Welan Gum (K1A96) und Xanthan-Gummi (Xantural 180^{®}) ist.

23. Darreichungsform zur Anwendung gemäß einem der Ansprüche 16 bis 22, **dadurch gekennzeichnet**, das die Komponente (c) wenigstens ein Opioid-Antagonist ist.

24. Darreichungsform zur Anwendung gemäß einem der Ansprüche 16 bis 23, **dadurch gekennzeichnet, dass** das Emetikum gemäß Komponente (d) auf einem oder mehreren Inhaltsstoffen von Radix Ipecacuanhae (Brechwurzel), vorzugsweise auf dem Inhaltsstoff Emetin basiert, und/oder Apomorphin ist.

25. Darreichungsform zur Anwendung gemäß einem der Ansprüche 16 bis 24, **dadurch gekennzeichnet, dass** die Komponente (e) wenigstens ein physiologisch verträglicher Farbstoff ist.

26. Darreichungsform zur Anwendung gemäß einem der Ansprüche 16 bis 25, **dadurch gekennzeichnet, dass** die Komponente (f) wenigstens ein Bitterstoff ausgewählt aus der Gruppe umfassend Aromaöle, vorzugsweise Pfefferminzöl, Eukalyptusöl, Bittermandelöl, Menthol und deren Mischungen, Fruchtaromastoffe, vorzugsweise von Zitronen, Orangen, Limonen, Grapefruit und deren Mischungen aus wenigstens 2 Komponenten, Denatoniumbenzoat und deren Mischungen aus wenigstens 2 Komponenten ist.

27. Darreichungsform zur Anwendung gemäß einem der Ansprüche 16 bis 26, **dadurch gekennzeichnet, dass** der Wirkstoff (A) von der Komponente (c) und/oder (d) und/oder (f) räumlich getrennt, vorzugsweise ohne direkten Kontakt vorliegt, wobei der Wirkstoff bzw. die Wirkstoffe (A) bevorzugt in wenigstens einer Untereinheit (X) und die Komponenten (c) und/oder (d) und/oder (f) in wenigstens einer Untereinheit (Y) vorliegen und die Komponenten (c) und/oder (d) und/oder (f) aus der Untereinheit (Y) bei bestimmungsgemäßer Applikation der Darreichungsform im Körper bzw. bei Einnahme nicht ihre Wirkung entfalten.

## Claims

1. An oral dosage form safeguarded against abuse with controlled opioid release for use in pain management for a period of at least 24 hours, **characterized in that** it is **in the form of a tablet,** comprises at least one opioid with potential for abuse (A) and/or one of the physiologically acceptable compounds thereof, at least one synthetic or natural polymer (C), optionally at least one delayed-release matrix material, optionally at least one delayed-release coating, optionally at least one physiologically acceptable excipient (B) and optionally at least one wax (D), wherein component (C) or (D) each has a breaking strength of at least 500 N, component(s) (C) and optionally (D) being present in quantities such that the dosage form exhibits a breaking strength of at least 500 N, and wherein the administration is once daily;
and wherein the breaking strength of component (C) or (D) is determined as follows: the material is press-moulded into a tablet with a diameter of 10 mm and a height of 5 mm with a force of 150 N, at a temperature corresponding to at least the softening point, determined by means of a DSC diagram of the material; the tablets produced in this way are used to determine the breaking strength according to the method for determining the breaking strength of tablets, published in the European Pharmacopoeia 1997, page 143, 44, method no. 2.9.8, using the apparatus listed below; a Zwick material testing machine "Zwick Z 2.5", material testing machine Fmax 2.5 kN with a traverse path of max. 1150 mm, which is to be set by means of a structure using a column and a spindle, a free working space to the rear of 100 mm, and a test speed adjustable between 0.1 to 800 mm/min and software: testControl is used as the apparatus for the measurement; a compression die with screwable inserts and a cylinder, diameter 10 mm, a force transducer, Fmax. 1 kN, diameter 8 mm, class 0.5 from 10 N, class 1 from 2 N according to ISO 7500-1, with manufacturer's test certificate M according to DIN 55350-18, Zwick gross force Fmax 1.45 kN are used for measurement.

2. The dosage form for use according to claim 1, **characterized in that** the opioid is at least one opioid selected from the group comprising oxycodone, hydromorphone, morphine, oxymorphone, tramadol, the stereoisomers thereof, the racemates thereof, the enantiomers thereof, the diastereomers thereof in any mixtures, the physiologically acceptable compounds thereof, preferably physiologically acceptable salts, very particularly preferably hydrochlorides and solvates, and the derivatives thereof, preferably esters, ethers or amides.

3. The dosage form for use according to claim 1, **characterized in that** the opioid present is at least one opioid selected from the group comprising (2R,3R)-1-dimethylamino-3-(3-methoxyphenyl)-2-methylpentan-3-ol, (1RS,3RS,6RS)-6-dimethylaminomethyl-1-(3-methoxyphenyl)cyclohexane- 1,3-diol, (1R,2R)-3-(2-dimethylaminomethylcyclohexyl)-phenol, the physiologically acceptable salts thereof, preferably hydrochlorides, physiologically acceptable enantiomers, stereoisomers, diastereomers and racemates and the physiologically acceptable derivatives thereof, preferably ethers, esters or amides.

4. The dosage form for use according to any one of claims 1 to 3, **characterized in that** the polymer (C) is at least one polymer selected from the group comprising polyalkylene oxides, polyethylenes, polypropylenes, polyvinyl chlorides, polycarbonates, polystyrenes, poly(meth)acrylates, the copolymers thereof and mixtures of at least two representatives of the stated polymer classes or polymers.

5. The dosage form for use according to claim 4, **characterized in that** the polyalkylene oxide is a polymethylene oxide, polyethylene oxide or polypropylene oxide.

6. The dosage form for use according to any one of claims 1 to 5, **characterized in that** the polymer (C) is a high molecular weight polyethylene oxide.

7. The dosage form for use according to any one of claims 1 to 6, **characterized in that** the polymer (C) is a water-soluble or water-swellable polymer.

8. The dosage form for use according to any one of claims 1 to 7, **characterized in that** the polyethylene oxide (C) has a molecular weight of at least 0.5 million.

9. The dosage form for use according to claim 8, **characterized in that** the molecular weight of the polyethylene oxide (C) is 1-15 million.

10. The dosage form for use according to any one of claims 1 to 9, **characterized in that** at least one natural, semisynthetic or synthetic wax with a softening point of at least 60 °C is present as the wax (D).

11. The dosage form for use according to claim 10, **characterized in that** the wax (D) is carnauba wax or beeswax.

12. The dosage form for use according to any one of claims 1 to 11, **characterized in that** the opioid is present in a delayed-release matrix.

13. The dosage form for use according to claim 12, **characterized in that** component (C) and/or component (D) also serve/s as a delayed-release matrix component.

14. The dosage form for use according to any one of claims 1 to 13, **characterized in that** at least one excipient (B) serves as a material for the delayed-release matrix.

15. The dosage form for use according to any one of claims 1 to 14, **characterized in that** it has a coating, preferably a delayed-release coating.

16. The dosage form for use according to any one of claims 1 to 15, **characterized in that** it has at least one of the following further abuse-preventing components (a)-(f) as the excipient (B):
(a) at least one substance that irritates the nasal passages and/or pharynx,
(b) at least one viscosity-increasing agent which, with the assistance of a necessary minimum quantity of an aqueous liquid, preferably as an extract obtained from the dosage form, forms a gel which preferably remains visually distinguishable when introduced into a further quantity of an aqueous liquid,
(c) at least one antagonist for the active ingredient with potential for abuse,
(d) at least one emetic,
(e) at least one dye as an aversive agent,
(f) at least one bitter substance.

17. The dosage form for use according to claim 16, **characterized in that** the component (a) irritant causes burning, itching, an urge to sneeze, increased formation of secretions or a combination of at least two of these stimuli.

18. The dosage form for use according to claim 16 or 17, **characterized in that** the component (a) irritant is based on one or more constituents of at least one hot substance drug.

19. The dosage form for use according to claim 18, **characterized in that** the hot substance drug is at least one drug selected from the group comprising Allii sativi bulbus, Asari rhizoma c. herba, Calami rhizoma, Capsici fructus (capsicum), Capsici fructus acer (cayenne pepper), Curcumae longae rhizoma, Curcumae xanthorrhizae rhizoma, Galangae rhizoma, Myristicae semen, Piperis nigri fructus (pepper), Sinapis albae semen (white mustard seed), Sinapis nigri semen, Zedoariae rhizoma and Zingiberis rhizoma, particularly preferably at least one drug selected from the group comprising Capsici fructus (capsicum), Capsici fructus acer (cayenne pepper) and Piperis nigri fructus (pepper).

20. The dosage form for use according to claim 18 or 19, **characterized in that** the constituent of the hot substance drug is present as an o-methoxy(methyl)phenol compound, an acid amide compound, a mustard oil or a sulfide compound or is derived from such a compound.

21. The dosage form for use according to any one of claims 18 to 20, **characterized in that** the constituent of the hot substance drug is at least one constituent selected from the group comprising myristicin, elemicin, isoeugenol, β-asarone, safrole, gingerols, xanthorrhizol, capsaicinoids, preferably capsaicin, piperine, preferably trans-piperine, glucosinolates, preferably based on non-volatile mustard oils, particularly preferably based on p-hydroxybenzyl mustard oil, methylmercapto mustard oil or methylsulfonyl mustard oil, and a compound derived from these constituents.

22. The dosage form for use according to any one of claims 16 to 21, **characterized in that** component (b) is at least one viscosity-increasing agent selected from the group comprising microcrystalline cellulose with 11% by weight carboxymethylcellulose sodium (Avicel^{®} RC 591), carboxymethylcellulose sodium (Blanose^{®}, CMC-Na C300P^{®},Frimulsion BLC-5^{®}, Tylose C300 P^{®}), polyacrylic acid (Carbopol^{®} 980 NF, Carbopol^{®} 981), locust bean flour (Cesagum^{®} LA-200, Cesagum^{®} LID/150, Cesagum^{®} LN-1), pectins from citrus fruits or apples (Cesapectin^{®} HM Medium Rapid Set), waxy maize starch (C^{∗}Gel 04201^{®}), sodium alginate (Frimulsion ALG (E401)^{®}), guar flour (Frimulsion BM^{®}, Polygum 26/1-75^{®}), iota-carrageenan (Frimulsion D021^{®}), karaya gum, gellan gum (Kelcogel F^{®}, Kelcogel LT100^{®}), galactomannan (Meyprogat 150 ^{®}), tara stone flour (Polygum 43/1^{®}), propylene glycol alginate (Protanal-Ester SD-LB^{®}), apple pectin, sodium hyaluronate, tragacanth, tara gum (Vidogum SP 200^{®}), fermented polysaccharide welan gum (K1A96) and xanthan gum (Xantural 180^{®}).

23. The dosage form for use according to any one of claims 16 to 22, **characterized in that** component (c) is at least one opioid antagonist.

24. The dosage form for use according to any one of claims 16 to 23, **characterized in that** the component (d) emetic is based on one or more constituents of ipecacuanha (ipecac) root, preferably based on the constituent emetine, and/or is apomorphine.

25. The dosage form for use according to any one of claims 16 to 24, **characterized in that** component (e) is at least one physiologically acceptable dye.

26. The dosage form for use according to any one of claims 16 to 25, **characterized in that** component (f) is at least one bitter substance selected from the group comprising aromatic oils, preferably peppermint oil, eucalyptus oil, bitter almond oil, menthol and mixtures thereof, fruit aroma substances, preferably from lemons, oranges, limes, grapefruit and mixtures thereof of at least 2 components, denatonium benzoate and mixtures thereof of at least 2 components.

27. The dosage form for use according to any one of claims 16 to 26, **characterized in that** the active ingredient (A) is spatially separated, preferably without direct contact, from component (c) and/or (d) and/or (f), wherein the active ingredient(s) (A) is/are preferably present in at least one subunit (X) and the components (c) and/or (d) and/or (f) is/are present in at least one subunit (Y), and, when the dosage form is correctly administered, components (c) and/or (d) and/or (f) from subunit (Y) do not exert their effect in the body or on taking.

## Revendications

1. Forme posologique orale garantie contre les abus, à libération contrôlée d'opioïde pour une utilisation analgésique sur une période d'au moins 24 heures, **caractérisée en ce qu'**elle se présente **sous forme d'un comprimé**, qu'elle comprend au moins un opioïde à potentiel d'abus (A) et/ou l'un de ses composés physiologiquement compatibles, au moins un polymère synthétique ou naturel (C), éventuellement au moins un matériau matriciel retardateur, éventuellement au moins un enrobage retardateur, éventuellement au moins un excipient physiologiquement compatible (B) et éventuellement au moins une cire (D), dans laquelle le composant (C) ou (D) a respectivement une résistance à la rupture d'au moins 500 N, dans laquelle le(s) composant(s) (C) et éventuellement (D) est (sont) présent(s) dans des quantités telles que la forme posologique a une résistance à la rupture d'au moins 500 N, et dans laquelle l'administration s'effectue une fois par jour ;
et dans laquelle la résistance à la rupture du composant (C) ou (D) est déterminée comme suit : le matériau est pressé sous la forme d'un comprimé d'un diamètre de 10 mm et d'une hauteur de 5 mm avec une force de 150 N, à une température correspondant au moins au point de ramollissement déterminé à l'aide de la courbe DSC du matériau ; la résistance à la rupture des comprimés ainsi préparés est conforme au procédé définissant la résistance à la rupture des comprimés, publiée dans la Pharmacopée européenne en 1997, page 143, 44, procédé n°2.9.8. en utilisant les appareils listés ci-dessous ; une machine d'essai de matériaux Zwick « Zwick Z 2.5 » utilisée comme appareil de mesure, une machine d'essai de matériaux de Fmax de 2,5 kN avec une course transversale de 1150 mm maximum, qui doit être réglée au moyen d'une structure utilisant une colonne et une broche, un espace de travail libre vers l'arrière de 100 mm et avec une vitesse ajustable allant de 0,1 à 800 mm/min. et un programme : testControl sont utilisés ; un tampon de pression avec inserts vissables et un cylindre, d'un diamètre de 10 mm, un capteur de force, Fmax de 1 kN, d'un diamètre de 8 mm, de catégorie 0,5 à partir de 10 N, de catégorie 1 à partir de 2 N, conforme à ISO 7500-1, avec un certificat M de production conformément à la norme DIN 55350-18 et la force brute de Zwick de Fmax de 1,45 kN sont utilisé pour la mesure.

2. Forme posologique pour une utilisation selon la revendication 1, **caractérisée en ce que** l'opioïde est au moins un opioïde choisi dans le groupe comprenant l'oxycodone, l'hydromorphone, la morphine, l'oxymorphone, le tramadol, leurs stéréoisomères, leurs racémates, leurs énantiomères, leurs diastéréoisomères en mélanges quelconques, leurs composés physiologiquement compatibles, de préférence leurs sels physiologiquement compatibles, tout particulièrement de préférence les chlorhydrates et les solvates, et leurs dérivés, de préférence les esters, les éthers ou les amides.

3. Forme posologique pour une utilisation selon la revendication 1, **caractérisée en ce que** l'opioïde présent est au moins un opioïde choisi dans le groupe comprenant (2R, 3R)-1-diméthylamino-3-(3-méthoxyphényl)-2-méthylpentane-3-ol, (1RS,3RS,6RS)-6-diméthylaminométhyl-1-(3-méthoxyphényl)-cyclohexane-1,3-diol, (1R, 2R)-3-(2-diméthylaminométhyl-cyclohexyl)-phénol, leurs sels physiologiquement compatibles, de préférence les chlorhydrates, les énantiomères physiologiquement compatibles, les stéréoisomères, les diastéréoisomères et les racémates et leurs dérivés physiologiquement compatibles, de préférence les éthers, les esters ou les amides.

4. Forme posologique pour une utilisation selon l'une des revendications 1 à 3, **caractérisée en ce que** le polymère (C) est au moins un polymère choisi dans le groupe comprenant les polyoxydes d'alkylène, les polyéthylènes, les polypropylènes, les polychlorures de vinyle, les polycarbonates, les polystyrènes, les poly(méth)acrylates, leurs copolymères et mélanges constitués d'au moins deux représentants des catégories de polymère(s) mentionné(s).

5. Forme posologique pour une utilisation selon la revendication 4, **caractérisée en ce que** le polyoxyde d'alkylène est un polyoxyde de méthylène, un oxyde de polyéthylène ou un oxyde de polypropylène.

6. Forme posologique pour une utilisation selon l'une des revendications 1 à 5, **caractérisée en ce qu'**un oxyde de polyéthylène macromoléculaire est présent comme polymère (C).

7. Forme posologique pour une utilisation selon l'une des revendications 1 à 6, **caractérisée en ce que** le polymère (C) est un polymère hydrosoluble ou hydrogonflable.

8. Forme posologique pour une utilisation selon l'une des revendications 1 à 7, **caractérisée en ce que** l'oxyde de polyéthylène (C)a un poids moléculaire d'au moins 0,5 million.

9. Forme posologique pour une utilisation selon la revendication 8, **caractérisée en ce que** le poids moléculaire de l'oxyde de polyéthylène (C) est de 1 à 15 millions.

10. Forme posologique pour une utilisation selon l'une des revendications 1 à 9, **caractérisée en ce que** la cire (D) présente est au moins une cire naturelle, semi-synthétique ou synthétique ayant un point de ramollissement d'au moins 60°C.

11. Forme posologique pour une utilisation selon la revendication 10, **caractérisée en ce que** la cire (D) est une cire de carnauba ou une cire d'abeille.

12. Forme posologique pour une utilisation selon l'une des revendications 1 à 11, **caractérisée en ce que** l'opioïde est présent sous la forme d'une matrice retardatrice.

13. Forme posologique pour une utilisation selon la revendication 12, **caractérisée en ce que** le composant (C) et/ou le composant (D) servent aussi de composant matriciel retardateur.

14. Forme posologique pour une utilisation selon l'une des revendications 1 à 13, **caractérisée en ce qu'**au moins un excipient (B) sert de matériau matriciel retardateur.

15. Forme posologique pour une utilisation selon l'une des revendications 1 à 14, **caractérisée en ce qu'**elle a un enrobage, de préférence un enrobage retardateur.

16. Forme posologique pour une utilisation selon l'une des revendications 1 à 15, **caractérisée en ce qu'**elle a comme excipient (B) au moins un des autres composants empêchant les abus suivants (a)-(f) :
(a) au moins une substance irritante pour le nez et/ou le pharynx,
(b) au moins un agent augmentant la viscosité qui, à l'aide d'une quantité minimale nécessaire d'un liquide aqueux, de préférence sous la forme d'un extrait obtenu à partir de la forme posologique, forme un gel qui peut être distingué visuellement de préférence lors de son introduction dans une quantité supplémentaire d'un liquide aqueux,
(c) au moins un antagoniste de l'agent actif à potentiel d'abus,
(d) au moins un émétique
(e) au moins un colorant comme agent aversif,
(f) au moins une substance amère.

17. Forme posologique pour une utilisation selon la revendication 16, **caractérisée en ce que** les substances irritantes selon le composant (a) provoquent une brûlure, une démangeaison, une envie d'éternuer, une sécrétion accrue ou une combinaison d'au moins deux de ces stimuli.

18. Forme posologique pour une utilisation selon la revendication 16 ou 17, **caractérisée en ce que** les substances irritantes selon le composant (a) sont basées sur un ou plusieurs ingrédients d'au moins une drogue piquante.

19. Forme posologique pour une utilisation selon la revendication 18, **caractérisée en ce que** la drogue piquante est au moins une drogue choisie dans le groupe comprenant Allii sativi Bulbus, Asari Rhizoma c. Herba, Calami Rhizoma, Capsici Fructus (piment), Capsici Fructus acer (piment de Cayenne), Curcumae longae Rhizoma, Curcumae xanthorrhizae Rhizoma, Galangae Rhizoma, Myristicae Semen, Piperis nigri Fructus (poivre), Sinapis albae (Erucae) Semen, Sinapis nigri Semen , Zedoariae Rhizoma et Zingiberis Rhizoma, de manière particulièrement préférée au moins une drogue choisie dans le groupe comprenant Capsici fructus (piment), Capsici fructus acer (piment de Cayenne) et Piperis nigri fructus (poivre).

20. Forme posologique pour une utilisation selon la revendication 18 ou 19, **caractérisée en ce que** l'ingrédient de la drogue piquante se présente sous la forme d'un composé o-méthoxy(méthyl)phénol, d'un composé amide d'acide, d'une huile de moutarde ou d'un composé sulfuré ou est dérivé d'un tel composé.

21. Forme posologique pour une utilisation selon l'une des revendications 18 à 20, **caractérisée en ce que** l'ingrédient de la drogue piquante est au moins un ingrédient choisi dans le groupe comprenant la myristicine, l'élémicine, l'isoeugénol, la β-asarone, le safrole, l'huile de gingembre, le xanthorrhizol, les capsaïcinoïdes , de préférence la capsaïcine, la pipérine, de préférence la trans-pipérine, les glucosinolates, de préférence à base d'huiles de moutarde non volatiles, de manière particulièrement préférée à base d'huile de moutarde p-hydroxybenzyl, d'huile de moutarde méthylmercaptose ou d'huile de moutarde méthylsulfonyl, et un composé dérivé de ces ingrédients.

22. Forme posologique pour une utilisation selon l'une des revendications 16 à 21, **caractérisée en ce que** le composant (b) est au moins un agent augmentant la viscosité choisi dans le groupe comprenant la cellulose microcristalline avec 11 % en poids de carboxyméthylcellulose de sodium (Avicel^{®} RC 591), la carboxyméthylcellulose de sodium (Blanose^{®}, CMC-Na C300P^{®}, Frimulsion BLC-5^{®}, Tylose C300 P^{®}), acide polyacrylique (Carbopol^{®} 980 NF, Carbopol^{®} 981), gomme de caroube (Cesagum^{®} LA-200, Cesagum^{®} LID/ 150, Cesagum^{®} LN-1), pectines d'agrumes ou de pommes (Cesapectin^{®} HM Medium Rapid Set), amidon de maïs waxy (C*Gel 04201^{®}), alginate de sodium (Frimulsion ALG (E401)^{®}), gomme de guar (Frimulsion BM^{®}, Polygum 26/1-75 ^{®}), iota-carraghénane (Frimulsion D021^{®}), gomme karaya, gomme gellane (Kelcogel F^{®}, Kelcogel LT100^{®}), galactomannane (Meyprogat 150^{®}), gomme tara (Polygum 43/1^{®}), propylène glycoalginate (protanal ester SD-LB ^{®}), pectine de pomme, hyaluronate de sodium, astragale, gomme tara (Vidogum SP 200^{®}), Gomme Welan en polysaccharide fermenté (K1A96) et Gomme Xanthane (Xantural 180^{®}).

23. Forme posologique pour une utilisation selon l'une des revendications 16 à 22, **caractérisée en ce que** le composant (c) est au moins un opioïde antagoniste.

24. Forme posologique pour une utilisation selon l'une des revendications 16 à 23, **caractérisée en ce que** l'émétique selon le composant (d) est à base d'un ou plusieurs ingrédients de Radix Ipecacuanhae (racine d'ipéca), de préférence de l'ingrédient émétine, et/ou est l'apomorphine.

25. Forme posologique pour une utilisation selon l'une des revendications 16 à 24, **caractérisée en ce que** le composant (e) est au moins un colorant physiologiquement compatible.

26. Forme posologique pour une utilisation selon l'une des revendications 16 à 25, **caractérisée en ce que** le composant (f) est au moins une substance amère choisie dans le groupe comprenant les huiles aromatiques, de préférence l'huile de menthe poivrée, l'huile d'eucalyptus, l'huile d'amande amère, le menthol et leurs mélanges, les arômes de fruits, de préférence le citron, l'orange, le citron vert, le pamplemousse et leurs mélanges d'au moins 2 composants, le benzoate de dénatonium et leurs mélanges d'au moins 2 composants.

27. Forme posologique pour une utilisation selon l'une des revendications 16 à 26, **caractérisée en ce que** l'agent actif (A) est spatialement séparé du composant (c) et/ou (d) et/ou (f), de préférence sans contact direct, dans laquelle l'agent actif ou les agents actifs (A) sont de préférence présents dans au moins un sous-ensemble (X) et les composants (c) et/ou (d) et/ou (f) dans au moins un sous-ensemble (Y) et les composants (c) et/ou (d) et/ou (f) du sous-ensemble (Y) ne produisent pas leur effet lors de l'application conforme à la forme posologique dans le corps ou lors de l'ingestion.
